# EUROPEAN PATENT SPECIFICATION

(11) **EP 3 454 904 B1**
(45) Date of publication and mention of the grant of the patent: **14.12.2022**
(21) Application number: 17728073.2
(22) Date of filing: 15.05.2017
(51) Int. Cl.: A61K 45/00, A61P 25/28

(54) **INHIBITION OF BETA-2 NICOTINIC ACETYLCHOLINE RECEPTORS TO TREAT ALZHEIMER'S DISEASE PATHOLOGY**
HEMMUNG VON BETA-2-NICOTINISCHEN ACETYLCHOLINREZEPTOREN ZUR BEHANDLUNG VON MORBUS ALZHEIMER
INHIBITION DE RÉCEPTEURS NICOTINIQUE BÊTA-2 DE L'ACÉTYLCHOLINE POUR TRAITER UNE PATHOLOGIE DE LA MALADIE D'ALZHEIMER

(30) Priority: 13.05.2016 US 201662336428 P
(43) Date of publication of application: 20.03.2019
(73) Proprietor: Institut Pasteur, 75015 Paris (FR)
(72) Inventor: MASKOS, Uwe, 75007 Paris (FR); LOMBARDO, Sylvia, Civitavecchia, RM 00053 (IT); CATTEAU, Julie, 87110 Solignac (FR); BESSON, Morgane, 92160 Antony (FR); KOUKOULI, Fani, 75015 Paris (FR)
(74) Representative: Regimbeau
(86) International application number: PCT/EP2017/061630
(87) International publication number: WO 2017/194789

(56) References cited:
- WO-A1-2010/088400
- WO-A2-2012/068553
- SAMARD ERKULWATER: "Amantadine and the End-Stage Dementia of Alzheimer's Type", SOUTHERN MEDICAL JOURNAL,, vol. 82, no. 5, 1 May 1989 (1989-05-01), pages 550-554, XP009194964,

## Description

### SUMMARY OF THE INVENTION

It is to be understood in this application that any reference to a method of treatment of an animal or human body is to be regarded as referring to a composition for use in such a method.

This application provides compositions for use in methods of reducing development of mild cognitive impairment and/or learning disorders and/or memory disorders in a subject suspected to have or suffering from Alzheimer's disease (AD). Said methods comprise administering a therapeutically effective amount of a beta-2 nicotinic acetylcholine receptor (nAChR) antagonist and/or beta-2 nAChR negative allosteric modulator (NAM) to the subject, preferably during a limited period of time. Said beta-2 nAChR antagonist and/or beta-2 nAChR NAM reduces acetylcholine signaling through beta-2-containing nAChR(s) selected from the group consisting of: alpha-2 alpha-5 beta-2, alpha-2 beta-2, alpha-3 beta-2, alpha-4 beta-2, alpha-6 beta-2, alpha-7 beta-2, alpha-3 alpha-5 beta-2, alpha-3 alpha-6 beta-2, alpha-4 alpha-5 beta-2, alpha-4 beta-2 beta-3 and alpha-5 alpha-6 beta-2, preferably selected from the group consisting of : alpha-2 alpha-5 beta-2, alpha-3 beta-2 and alpha-2 beta-2; and more preferably through alpha-2 beta-2.

In some embodiments of the methods, the beta-2 nAChR antagonist and/or beta-2 nAChR NAM reduces acetylcholine signaling through at least one beta-2-containing nAChR.

In some embodiments of the methods, the beta-2 nAChR antagonist and/or beta-2 nAChR NAM reduces acetylcholine signaling through beta-2-containing nAChRs containing at least one, preferably two or three beta-2 subunits, and more preferably two beta-2 subunits.

In some embodiments of the methods, the subject is provided not only with beta-2 nAChRs antagonists or modulators, but also with an alpha-7 nAChR antagonist and/or alpha-7 nAChR NAM, such as S76892, EVP-6124 and MLA.

In some embodiments of the methods, the mild cognitive impairment and/or learning disorders and/or memory disorders are correlated to the beginning of a neurodegeneration.

In some embodiments of the methods, the neurodegeneration comprises loss of function of hippocampal interneurons.

In some embodiments of the methods, the subject is diagnosed with predementia phase or dementia phase of AD.

In some embodiments of the methods, the therapeutic dosing period is at least one month. In some embodiments, the therapeutic dosing period is at least six months, at least nine months, at least twelve months, at least eighteen months, or at least two, five, eight, ten, fifteen, twenty, twenty-five, thirty, thirty-five, forty years.

In some embodiments of the methods, the beta-2 nAChR antagonist and/or beta-2 nAChR NAM, optionally along with the alpha-7 nAChR antagonist and/or the alpha-7 nAChR NAM, is administered to the subject at a regular dosing interval of from three to twenty-four hours.

### DESCRIPTION OF THE PRIOR ART

AD is a progressive neurodegenerative disease, characterized by the spread of hallmark lesions, which are amyloid extracellular plaques and intracellular neurofibrillary tau tangles. Although AD has widespread effects throughout the brain, only a few neural systems are specifically involved and show consistent pathological changes (Braak and Braak, 1991). Notably, the cholinergic pathway, and cholinergic neurons located in the basal forebrain in particular, are highly subjected to degeneration in AD (Auld et al., 2002; Whitehouse et al., 1982). These basal forebrain neurons provide the primary cholinergic innervation to the neocortex, hippocampus, and other limbic regions (Woolf and Butcher, 2011). Moreover, AD has also been consistently associated with a considerable loss of high affinity nAChRs that contain the beta-2 subunit (Gotti et al., 2006). This loss seems to occur in early stages of AD and to be region-specific. Evidence exists also for altered alpha-7 containing nAChRs in AD patients, notably in their hippocampus (Nordberg, 2001). nAChRs are crucially implicated in cognitive processing and have been proposed to significantly contribute to the cognitive deterioration observed in AD patients, probably in association with amyloid pathology (Okada et al., 2013).

Previous studies extensively reported degeneration of the cholinergic system in AD, particularly the basal forebrain cholinergic cells that innervate the neocortex and hippocampus. Their degeneration in AD is associated with reduced levels of pre- and postsynaptic cholinergic markers in the cortex, hippocampus, and amygdala.

Yet, the role of specific subunits of nAChRs in memory dysfunction caused by amyloid pathology is still unknown. In particular, the role of the beta-2 subunit of nAChRs on AD etiology has never been addressed so far.

There is therefore a need in the art to further understand the signaling pathways that underly development of AD pathology and in turn to identify modulators that interfere with such signaling pathways in order to protect subjects suffering from pre-dementia or early stage AD from developing AD pathology and/or treat them.

### DETAILED DESCRIPTION OF THE INVENTION

The examples of this application use a mouse obtained with the use of lentiviral and AAV vectors that express the mutated form of the human Amyloid Precursor Protein (hAPP) harbouring three pathogenic mutations: Swedish, London and Austrian (hAPP-SLA). The role of nAChRs in memory dysfunction caused by amyloid pathology was characterized in these mouse models.

An analysis of the contribution of the alpha-7 and beta-2 nAChR subunits was conducted because an interaction between these subunits and Amyloid beta (Abeta) has been reported, mainly for the beta-2 subunit so far (Liu et al., 2012, 2009; Nordberg et al., 1995; Wang et al., 2000a, 2000b), reviewed in Lombardo and Maskos (Lombardo and Maskos, 2015). In addition, both alpha-7 and beta-2 nAChR subunits are widely expressed in the hippocampus (Jones and Yakel, 1997), and this brain structure receives cholinergic afferences from the basal forebrain (Woolf and Butcher, 2011).

The inventors assessed the role of these particular subunits in the onset of AD pathology by analyzing the effect of their vectors in mice that were deprived (knock-out, KO) of alpha-7 and/or beta-2 nAChR subunits.

First, the hAPP-SLA lentivirus was introduced into mice harboring null mutations for alpha-7 and/or beta-2 subunits and the contribution of the alpha-7 and/or beta-2 nAChR subunits to AD phenotypes was analyzed.

The lentiviral vector used by the Inventors targets the expression of hAPP-SLA in the dentate gyrus of the hippocampus, a brain region with a key role in memory formation (Morris, 2006; Squire et al., 2007) that is implicated in AD pathology (Ohm, 2007). Wild-type (WT) mice developed AD-like pathological features such as memory loss and Abeta deposition that was restricted to the targeted area.

As demonstrated in the Examples 1-9, the beta-2 subunit null mice are protected from the hAPP-SLA-induced pathology, which demonstrates that signaling through beta-2 nAChR subunit-containing nAChRs is required for development of the cognitive deficit in this system.

These data strongly suggest that the lack of the beta-2 subunit prevents Abeta -induced memory loss, since beta-2 KO animals are protected from the recognition memory impairment induced by APP-SLA expression in control mice. To date, this is the first *in vivo* demonstration of a role for the beta-2 subunit in amyloid pathology.

Similar results have been obtained when an AAV vector targeting cortical regions of the brain was used to express the mutated form of hAPP (examples 10-11).

Moreover, the results obtained by the present inventors show that double KO alpha-7 beta-2 animals expressing Abeta are completely normal. This indicates that blocking at the same time beta-2 and alpha-7 containing receptors is a promising means for impairing AD onset in the wild-type mouse.

### A. Definitions

Unless otherwise defined herein, scientific and technical terms used in connection with the present disclosure shall have the meanings that are commonly understood by those of ordinary skill in the art. Further, unless otherwise required by context, singular terms shall include the plural and plural terms shall include the singular. Generally, nomenclatures used in connection with, and techniques of, biochemistry, enzymology, molecular and cellular biology, microbiology, genetics and protein and nucleic acid chemistry and hybridization described herein are those well-known and commonly used in the art. In case of conflict, the present specification, including definitions, will control. The materials, methods, and examples are illustrative only and not intended to be limiting.

The methods and techniques of the present disclosure are generally performed according to conventional methods well known in the art and as described in various general and more specific references that are cited and discussed throughout the present specification unless otherwise indicated. See, e.g., Sambrook et al., Molecular Cloning: A Laboratory Manual, 3d ed., Cold Spring Harbor Laboratory Press, Cold Spring Harbor, N.Y. (2001); Ausubel et al., Current Protocols in Molecular Biology, Greene Publishing Associates (1992, and Supplements to 2002); Taylor and Drickamer, Introduction to Glycobiology, Oxford Univ. Press (2003); Worthington Enzyme Manual, Worthington Biochemical Corp., Freehold, N.J.; Handbook of Biochemistry: Section A Proteins, Vol I, CRC Press (1976); Handbook of Biochemistry: Section A Proteins, Vol II, CRC Press (1976); Essentials of Glycobiology, Cold Spring Harbor Laboratory Press (1999).

Before the present compositions, methods, and other embodiments are disclosed and described, it is to be understood that the terminology used herein is for the purpose of describing particular embodiments only and is not intended to be limiting. It must be noted that, as used in the specification and the appended claims, the singular forms "a," "an" and "the" include plural referents unless the context clearly dictates otherwise.

The term "comprising" as used herein is synonymous with "including" or "containing", and is inclusive or open-ended and does not exclude additional, unrecited members, elements or method steps.

As used herein, the term "*in vitro"* refers to events that occur in an artificial environment, e.g., in a test tube or reaction vessel, in cell culture, in a Petri dish, etc., rather than within an organism (e.g., animal, plant, or microbe).

As used herein, the term "*in vivo*" refers to events that occur within an organism (e.g., animal, plant, or microbe).

As used herein, "Alzheimer's disease" (AD) refers to a neurodegenerative disease characterized by (1) a clinical phenotype typically centred on the presence of a progressive dementia that includes episodic memory impairment as a defining feature and involvement of other cognitive domains or skills, and (2) specific neuropathological changes that usually include intraneuronal (neurofibrillary tangles) and extracellular parenchymal lesions (senile plaques), which are often accompanied by synaptic loss and vascular amyloid deposits (see Dubois B et al., Revising the definition of Alzheimer's disease: a new lexicon. 2010 Lancet Neurol. 9 (11), 1118-27). In the meaning of the present application, AD refers to the whole spectrum of the clinical phase of the disease and is not restricted to the dementia syndrome. It therefore encompasses both the predementia and dementia phases. The diagnosis is established *in vivo* and relies on a dual clinicobiological entity that requires the evidence of both specific memory changes and *in vivo* markers of Alzheimer's pathology that can include: CSF (cerebrospinal fluid) Abeta, total tau, and phospho-tau; retention of specific PET amyloid tracers; medial temporal lobe atrophy on magnetic resonance imaging ; and/or temporal/ parietal hypometabolism on fluorodeoxyglucose PET. Neurobiological changes responsible for AD include specific neuronal brain lesions (senile neuritic plaques and neurofibrillary tangles), neurodegeneration, neuronal/synaptic loss, and vascular amyloid deposits within the cerebral cortex. Additionally, two different stages can be distinguished: a predementia phase and a dementia phase of AD.

As used herein, the term "predementia phase of AD" refers to the early symptomatic, predementia stage of AD in which (1) clinical symptoms including episodic memory loss of the hippocampal type (characterised by a free recall deficit on testing not normalised with cueing) are present, but not sufficiently severe to affect instrumental activities of daily living and do not warrant a diagnosis of dementia; and in which (2) biomarker evidence from CSF or imaging is supportive of the presence of AD pathological changes (see Dubois B et al., Revising the definition of Alzheimer's disease: a new lexicon. 2010 Lancet Neurol. 9 (11), 1118-27).

As used herein, the term "dementia phase of AD" or "AD dementia" refers to the phase of AD during which cognitive symptoms are sufficiently severe to interfere with social functioning and instrumental activities of daily living, a threshold that is considered to define dementia in association with changes in episodic memory and in at least one other cognitive domain (see Dubois B et al., Revising the definition of Alzheimer's disease: a new lexicon. 2010 Lancet Neurol. 9 (11), 1118-27).

As used herein, the term "mild cognitive impairment" (MCI) refers to a heterogenous condition charaterised by mild cognitive changes associated with various underlying aetiologies. It applies to individuals with measurable MCI in the absence of a significant effect on instrumental activities of daily living. This diagnostic label is applied if there is no disease to which MCI can be attributed. It remains a term of exclusion for individuals who are suspected to have but do not meet the criteria for AD, in that they deviate from the clinicobiological phenotype of predementia stage of AD because they have memory symptoms that are not characteristic of AD or because they are biomarker negative. MCI also is a useful clinical designation for describing individuals who have a memory deficit characteristic of predementia phase of AD (i.e., amnestic syndrome of hippocampal type), but when biomarker evidence of Alzheimer's pathology is absent, uncertain, or testing has not been done. MCI also applies to individuals with mild cognitive impairment or memory disorders that are not consistent with predementia phase of AD, although biomarker evidence is present (see Dubois B et al., Revising the definition of Alzheimer's disease: a new lexicon. 2010 Lancet Neurol. 9 (11), 1118-27).

As used herein, the term "neurodegeneration" refers in general to progressive degeneration, death of neurons and degeneration/loss of synapses in the cerebral cortex and certain subcortical regions that is a characteristic pathological feature of AD.

As used herein, "neuron," "neuronal cell" and "neural cell" (including neural progenitor cells and neural stem cells) are used interchangeably to refer to nerve cells, i.e., cells that are responsible for conducting nerve impulses from one part of the body to another. Most neurons consist of three distinct portions: a cell body which contains the nucleus, and two different types of cytoplasmic processes: dendrites and axons. Dendrites, which are the receiving portion of the neuron, are usually highly branched, thick extensions of the cell body. The axon is typically a single long, thin process that is specialized to conducts nerve impulses away from the cell body to another neuron or muscular or glandular tissue. Axons may have side branches called "axon collaterals." Axon collaterals and axons may terminate by branching into many fine filaments called telodendria. The distal ends of telodendria are called synaptic end bulbs or axonal terminals, which contain synaptic vesicles that store neurotransmitters. Axons may be surrounded by a multilayered, white, phospholipid, segmented covering called the myelin sheath, which is formed by Schwann cells in the peripheral nervous system and oligodendrocytes in the central nervous system. Axons containing such a covering are "myelinated." "Axonogenesis" refers to the growth and differentiation of axonal processes by developing neurons. Neurons include sensory (afferent) neurons, which transmit impulses from receptors in the periphery to the brain and spinal cord and from lower to higher centers of the central nervous system. A neuron can also be motor (efferent) neurons which convey impulses from the brain and spinal cord to effectors in the periphery and from higher to lower centers of the central nervous system. Other neurons are association (connecting or interneuron) neurons which carry impulses from sensory neurons to motor neurons and are located within the central nervous system. The processes of afferent and efferent neurons arranged into bundles are called "nerves" when located outside the CNS or fiber tracts if inside the CNS. "Axon growth" or "axonal growth" includes axon extension, axon regeneration and axon elongation.

AD is characterized by learning impairment, and especially a progressive memory loss. In a small percentage, difficulties with language, executive functions, perception (agnosia), or execution of movements (apraxia) are more prominent than memory problems. AD does not typically affect all memory capacities equally. Older memories of the person's life (episodic memory), facts learned (semantic memory), and implicit memory (the memory of the body on how to do things, such as using a fork to eat) are often affected to a lesser degree than new facts or memories. Language problems may be characterised by a shrinking vocabulary and decreased word fluency, leading to a general impoverishment of oral and written language. In this stage, the person with AD is usually capable of communicating basic ideas adequately. While performing fine motor tasks such as writing, drawing or dressing, certain movement coordination and planning difficulties (apraxia) may be present, but they are commonly unnoticed.

As used herein, "reducing development of mild cognitive impairment and/or learning disorders and/or memory disorders in a subject suspected to have or suffering from AD" should be understood to encompass: ameliorating undesired symptoms associated with said disease; preventing manifestation of these symptoms before they occur; slowing down progression of said disease or symptoms; slowing down deterioration of said disease or symptoms; slowing down irreversible damage caused in a progressive (or chronic) stage of said disease; delaying onset of said disease symptoms; reducing severity of said disease; curing said disease; preventing said disease or symptoms from occurring altogether (for example in an individual generally prone to the disease) or a combination of any of the above. For example, in a subject suffering from memory impairment, for example as a result of AD, symptoms include deterioration of spatial short-term memory, memory recall and/or memory recognition, focused and sustained attention, learning, executive functions and/or mental flexibility.

As used herein, a "beta-2 nicotinic acetylcholine receptor" or a "beta-2 neuronal acetylcholine receptor" or a "β2 nicotinic acetylcholine receptor" or a "β2 neuronal acetylcholine receptor" or "CHRNB2" or "nAChRB2" is a nicotinic acetylcholine receptor (nAChR) comprising at least one, two or three subunits beta-2 (β2) subunit(s) (the Genbank accession number for the human cholinergic receptor nicotinic beta-2 subunit is NG_008027).

As used herein, a "alpha-7 nicotinic acetylcholine receptor" or a "alpha-7 neuronal acetylcholine receptor" or a "α7 nicotinic acetylcholine receptor" or a "α7 neuronal acetylcholine receptor" or "CHRNA7" or "nAChRA7" is a nicotinic acetylcholine receptor (nAChR) comprising at least one, two, three, four or five alpha-7 (α7) subunit(s) (the human cholinergic receptor nicotinic alpha-7 subunit is described in Sinkus et al, Neuropharmacology, 2015).

As used herein, an "orthosteric site" is the binding site/s on a nAChR macromolecule that is/are recognized by the endogenous agonist/s for that receptor.

As used herein, an "allosteric site" is the binding site/s on a nAChR macromolecule that is nonoverlapping and spatially distinct from, but conformationally linked to, the orthosteric binding site.

As used herein, an "orthosteric agonist" is a ligand that binds to the orthosteric site of a nAChR macromolecule and alters the receptor state, resulting in a signal transduction response downstream of the nAChR.

Responses can be measured by electrophysiology or imaging. Signaling pathways are discussed in Buckingham et al., Nicotinic acetylcholine receptor signalling: roles in Alzheimer's disease and amyloid neuroprotection. Pharmacol Rev. 2009 61(1):39-61.

As used herein, an "allosteric modulator" is a ligand that modifies the action of an orthosteric agonist by combining with an allosteric site on the nAChR macromolecule. A "positive allosteric modulator (PAM)" is an allosteric modulator that increases the action (affinity and/or efficacy) of an orthosteric agonist, whereas a "negative allosteric modulator (NAM)" is an allosteric modulator that decreases the action (affinity and/or efficacy) of an orthosteric agonist.

As used herein, a "beta-2 nicotinic acetylcholine receptor (nAChR) antagonist" is a molecule that binds to an orthosteric site of a beta-2 nAChR and disrupts the interaction and inhibits the function of an orthosteric agonist of said receptor.

As used herein, an "alpha-7 nicotinic acetylcholine receptor (nAChR) antagonist" is a molecule that binds to an orthosteric site of an alpha-7 nAChR and disrupts the interaction and inhibits the function of an orthosteric agonist of said receptor.

As used herein, "subject" means any mammal including mice or primates. In a preferred embodiment the subject is a human.

As used herein, the terms "treat," "treatment," "treating," and "amelioration" refer to therapeutic treatments, wherein the object is to reverse, alleviate, ameliorate, inhibit, slow down and/or stop the progression or severity of a condition associated with a disease or disorder. The terms include reducing or alleviating at least one adverse effect or symptom of a condition, disease or disorder. Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation of at least slowing of progress or worsening of symptoms that would be expected in absence of treatment. Beneficial or desired clinical results include, but are not limited to, alleviation of one or more symptom(s), diminishment of extent of disease, stabilized (i.e., not worsening) state of disease, delay or slowing of disease progression, amelioration or palliation of the disease state, and remission (whether partial or total), whether detectable or undetectable. The terms "treat," "treatment," "treating," and "amelioration" in reference to a disease also include providing relief from the symptoms or side-effects of the disease (including palliative treatment).

As used herein, the terms "prevent", "prevention", "preventing" refer to protective treatments, wherein the object is to impair, slow down, delay, reverse and/or stop the development of a condition associated with a disease or disorder, before said disease or disorders have been diagnosed.

As used herein, an "effective amount" is an amount of a chemical entity that is effective when administered following a dosing schedule over a therapeutic dosing period.

As used herein, the term "therapeutically effective amount" is an amount or dose of a chemical entity (such as a beta-2 nAChR antagonist or beta-2 nAChR NAM) that is effective to ameliorate, delay, or prevent any of the clinical signs, behaviors or events associated with MCI, learning disorders, memory disorders or AD. Alternatively, a therapeutically effective amount is sufficient to cause an improvement in a clinically significant condition in the individual.

As used herein, a "therapeutic dosing period" is a period of time during which a chemical entity is administered to a subject following a defined dosing schedule. In the meaning of the present application, a therapeutic dosing period refers to the period of time beginning with the administration of a first dose of a chemical entity (such as a beta-2 nAChR antagonist or beta-2 nAChR NAM), and ending with the administration of the last dose of said chemical entity on a continuing basis. For example, if the chemical entity is administered approximately every twelve hours for a total of nine doses, the therapeutic dosing period is approximately 96 hours, the time between the first dose and the ninth.

### B. Nicotinic Acetylcholine Receptors

Nicotinic acetylcholine receptors (nAChRs) are excitatory neurotransmitter receptors and members of the super-family of ligand-gated ion channels. The diversity of nAChRs and their nomenclature is well known in the literature. (See, e.g., Neil S. Millar, et al., "Diversity of vertebrate nicotinic acetylcholine receptors," Neuropharmacology 56 (2009) 237-246.) They are complex pentameric transmembrane proteins assembled from a diverse collection of subunits. The nomenclature of nAChR subunits has arisen in a somewhat arbitrary manner. Early biochemical studies of nAChRs purified from the electric organ of fish such as the marine ray Torpedo and the freshwater eel Electrophorus identified four protein subunits (for a detailed review, see Popot and Changeux, 1984). The four subunits were assigned the Greek letters α (alpha), β (beta), γ (gamma) and δ (delta) on the basis of their increasing apparent molecular weights when resolved on polyacrylamide gels. Of these four subunits, only the alpha subunit could be labelled by quaternary ammonium affinity-labelling reagents, which led to the conclusion that the alpha subunit was the principal agonist binding site. The subsequent molecular cloning of the Torpedo alpha subunit identified two adjacent cysteine residues (Cys192 and Cys193), which were believed to be important in agonist binding. The convention which has been adopted subsequently for the nomenclature of nAChR subunits is that nAChRs which contain two cysteine residues at positions analogous to Cys192 and Cys193 in the Torpedo alpha subunit have been classified as alpha-type subunits. Non-alpha subunits expressed at the vertebrate neuromuscular junction have been assigned the names beta-1, gamma, delta and epsilon, whereas non-alpha subunits expressed in the vertebrate nervous system have been assigned the names beta-2 - beta-4.

A long established convention is that nAChR subtypes are referred to by their subunit composition. For example, alpha-4 beta-2 refers to a nAChR subtype containing only alpha-4 and beta-2 subunits (even though the precise subunit stoichiometry of subunits within the receptor may not be known). Also, by convention, when the precise subunit composition of a nAChR subtype is unknown, this uncertainty is indicated by an asterisk (Lukas et al., 1999; Luetje, 2004). For example, alpha-4 beta-2 indicates a nAChR which is known to contain alpha-4 and beta-2 subunits but which may also contain additional subunit subtypes. In situations where both the subunit composition and also the subunit stoichiometry are known, the number of each subunit present in the assembled pentamer is indicated by subscript numbers (for example (alpha-1)₂ beta-1gamma delta).

In some embodiments, the beta-2 nAChR referred to in the present application has a subunit composition chosen from the group consisting of : alpha-2 alpha-5 beta-2, alpha-2 beta-2, alpha-3 beta-2, alpha-4 beta-2, alpha-6 beta-2, alpha-7 beta-2, alpha-3 alpha-5 beta-2, alpha-3 alpha-6 beta-2, alpha-4 alpha-5 beta-2, alpha-4 beta-2 beta-3 and alpha-5 alpha-6 beta-2; preferably selected from the group consisting of: alpha-2 alpha-5 beta-2, alpha-3 beta-2 and alpha-2 beta-2 ; and is more preferably from alpha-2 beta-2.

In some embodiments, the beta-2 nAChR referred to in the present application contains one, two or three beta-2 subunits, preferably two or three beta-2 subunits, and more preferably two beta-2 subunits.

Alpha-7 nAChRs are well known in the art. They are thoroughly described in Sinkus et al, Neuropharmacology 2015 and in Koukouli and Maskos, Biochemical Pharmacology 2015. Alpha-7 nAChRs often assemble as homomers of alpha-7 subunits only (Gotti et al, 2007). They can also assemble as pentameric heteromers, e.g., containing beta-2 subunit(s). They can also contain human duplicated alpha-7 subunits (dup α-7), referred to as CHRFAM7A as gene symbol. Such receptors are often relied on as alpha-7 dup-alpha-7 (α-7dup α-7) (Wang et al, 2014).

In some embodiments, the alpha-7 nAChRs referred to in the present application contains one, two, three, four or five alpha-7 subunits, preferably two, three or five alpha-7 subunits, and more preferably five alpha-7 subunits.

### C. Nicotinic Acetylcholine Receptor Antagonists

The methods of the invention can use any antagonist of the above-mentioned nAChRs.

Beta-2 nAChR antagonists are known in the art. Exemplary antagonists are disclosed in John W. Daly, "Nicotinic Agonists, Antagonists, and Modulators From Natural Sources," Cellular and Molecular Neurobiology, Vol. 25, Nos. 3/4, June 2005, 513-552. Exemplary antagonists include the *Erythrina* alkaloids Dihydro-beta-erythroidine, Erysodine, and Erythraline (which are selective for alpha-4 beta-2). Additional exemplary antagonists include (-)-7-methyl-2-exo-[3'-(6-[18F]fluoropyridin-2-yl)-5'-pyridinyl]-7-azabicyclo[2.2.1]heptane; 2-fluoro-3-(4-nitrophenyl)deschloroepibatidine; Coclaurine; Mecamylamine; alpha-Conotoxin; and Tubocurarine.

Preferred beta-2 nAChR antagonists that can be used in the context of the present invention are those listed in Table 1 below.

**Table 1 : Beta-2 nAChRs antagonists**

| **Molecule** | **Reaxys Registry Number** | **CAS Registry Number** | **Bibliographic Reference** |
|---|---|---|---|
| Dihydro-beta-erythroidine | 36193 | 29734-68-7 | Damaj et al (1995) |
| Erysodine | | 7290-03-1 | Decker et al (1995) |
| (-)-7-methyl-2-exo-[3'-(6-[18F]fluoropyridin-2-yl)-5'-pyridinyl]-7-azabicyclo[2.2.1]heptane | | | Kuwabara H, 2008 US 9284322 B2 |
| 2-fluoro-3-(4-nitrophenyl)deschloroepibatidine | | | Abdrakhmanova et al., 2006 |
| Coclaurine | 35607 | 2033-08-1 | Sowemimo BO, 1972 |
| Mecamylamine | 2715534 | 60-40-2; 6147-18-8; 107538-05-6; 107538-06-7 ; 826-39-1 | Rabenstein et al (2006) |
| | | | |
| Tubocurarine | 3898737 | 57-94-3 | Wotring and Yoon (1995) |

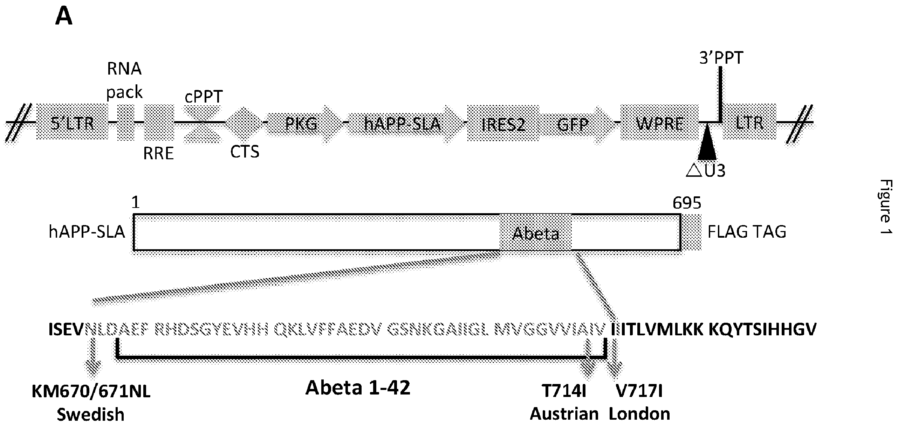

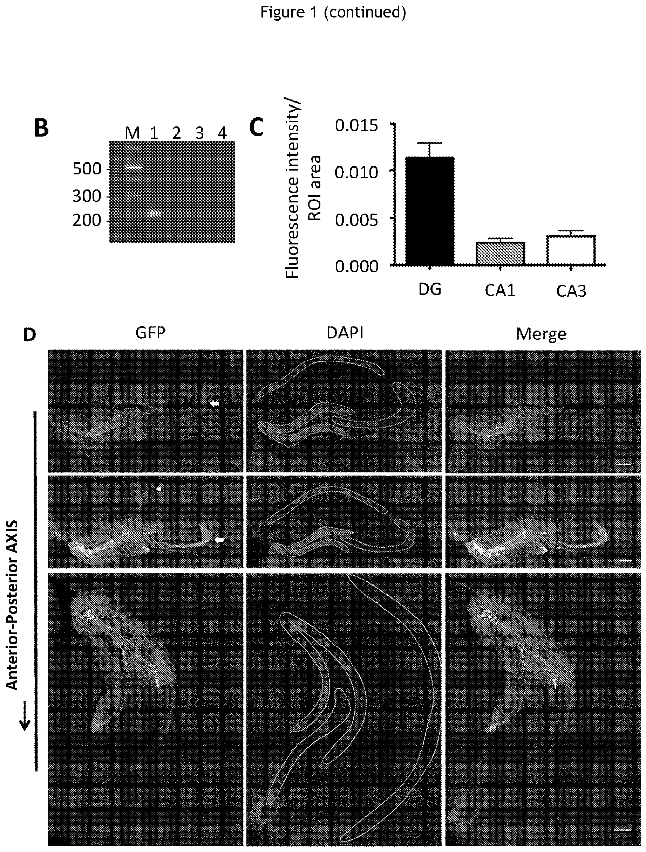

A particularly useful beta-2 antagonist is the cytisine-related molecule described in Figure 1 of Pouny et al, 2014, and having the following formula:

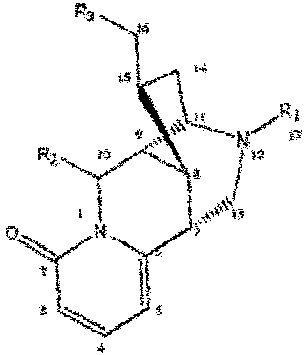

Another particularly useful beta-2 antagonist is dihydro-β-erythroidine hydrobromide (or "DHβE") which is commercially available at Tocris under the CAS number 29734-68-7. The structure of this antagonist is:

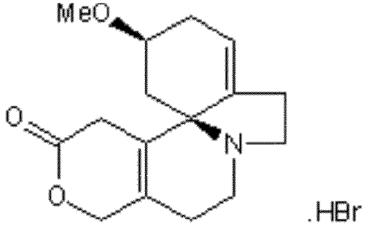

Alpha-7 nAChR antagonists are known in the art.

Preferred alpha-7 nAChR antagonists that can be used in the context of the present invention are listed in Table 2 below.

**Table 2 : Alpha-7 nAChRs antagonists**

| **Molecule** | **Reaxys Registery Number** | **CAS Registry Number** | **Bibliographic references** |
|---|---|---|---|
| Ethanol | | | Covernton PJO (1997) |
| | | | Yu D (1996) |
| Hydroxynorketamine | 30180525 | | Zanos Panos; (2016). |
| Ketamine | 2216965 | 6740-88-1 | |
| Kynurenic acid | 147451 ; 4018001 | 492-27-3 | Hilmas, C. (2001). |
| Lobeline | 91532 | 90-69-7 | Damaj, M. I (1997). |
| Methyllycaconitine (MLA) | 24141394 | 21019-30-7 | Sadigh-Eteghad S (2015). |
| Norketamine | 6274703 | 35211-10-0 | |
| Quinolizidine (-)-1-epi-207I | | | Tsuneki H, et al. (2004). |
| S76892 | | | Louis C, 2015 |
| dizocilpine (+)-MK-801 | 3651796 | 77086-21-6 | Briggs CA (1996). |

One of them is methyllycaconitine (MLA). It is commercially available (see Besson et al, PNAS 2007) and has the following structure :

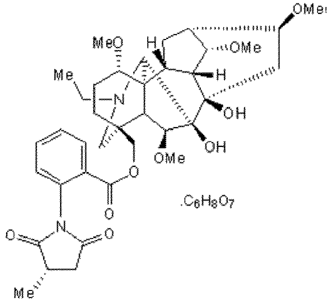

Identification and/or characterization of additional beta-2 or alpha-7 nAChR antagonists may be accomplished using *in vitro* and/or *in vivo* assays known in the art.

### D. Nicotinic Acetylcholine Receptor Negative Allosteric Modulators

The methods of the invention can use any negative allosteric modulators of the above-mentioned receptors.

In some embodiments, the methods of the invention use a negative allosteric modulator (NAM) which is specific for at least one beta-2 nAChR having a defined subunit composition (e.g., alpha-4 beta-2). In other embodiments, the NAM is active against at least two beta-2 nAChRs, said receptor having more than one type of subunit composition (e.g., alpha-4 beta-2 and alpha-7 beta-2). In some embodiments, the NAM is active against all beta-2 nAChRs.

In some embodiments, the methods of the invention use a NAM which is specific for a nAChR containing a least one, preferably two or three beta-2 subunits, and more preferably two beta-2 subunits.

Preferably, said beta-2 nAChR NAM reduces acetylcholine signaling through at least one (preferably two) beta-2-containing nAChR(s) selected from the group consisting of: alpha-2 alpha-5 beta-2, alpha-2 beta-2, alpha-3 beta-2, alpha-4 beta-2, alpha-6 beta-2, alpha-7 beta-2, alpha-3 alpha-5 beta-2, alpha-3 alpha-6 beta-2, alpha-4 alpha-5 beta-2, alpha-4 beta-2 beta-3 and alpha-5 alpha-6 beta-2, preferably selected from the group consisting of : alpha-2 alpha-5 beta-2, alpha-4 beta-2, alpha-3 beta-2 and alpha-2 beta-2 ; and more preferably through alpha-2 beta-2.

The invention may be practiced using a known beta-2 nAChR NAM. Examples of beta-2 nAChR NAMs are taught in the art. For example, Anshul Pandya, et al., "Allosteric modulators of the alpha-4 beta-2 subtype of neuronal nicotinic acetylcholine receptors," Biochemical Pharmacology 82 (2011) 952-958, describes known NAMs of nAChR alpha-4 beta-2, including, amantadine, memantine, steroids such as progesterone, and UCI-30002. (See also references cited therein.) Additional beta-2 nAChR NAMs are taught in Hugo R. Arias, et al., "Positive and Negative Regulation of Nicotinic Receptors," Adv. Protein Chem. Struct. Biol., 80 (2010) 153-203, and references cited therein. Examples include the methyllycaconitine derivative COB-3, the lead compound KAB-18 with specificity for the human alpha-4 beta-2 AChR, and UCI-30002 [N-(1,2,3,4-tetrahydro-1-naphthyl)-4-nitroaniline] with selectivity for the alpha-4 beta-2, alpha-7, and alpha-3 beta-4 AChRs over muscle AChRs. In addition to synthetic NAMs, several endogenous molecules and ions have been identified to possess the same basic pharmacological properties as synthetic NAMs, including fatty acids, the prototoxins, Lynx-1 and -2. Examples also include COB-1, COB-2, DDR-15, DDR-13, DDR-18, APB-8, DDR-1, DDR-2, KAB-21, DDR-19, KAB-24, KAB-22, KAB-23, KAB-19, KAB-20, KAB-30, IB-2, PPB-12, PPB-13, IB-4, COB-4, LYPD6, LYPD6B, PSCA, PATE-M, SLURP1, SLURP2, ly6A/E, ly6B-G, Ly6G6E, ly6H, SLEEPLESS, Odr-2, Boudin, Nl-lynx1, Nl-lynx2, Pr-lynx1, alpha-ctx, Erabutoxin b, Bucandin, Candoxin, Fasciculins, alpha-colubritoxin.

Preferred beta-2 nAChR NAMs that can be used in the context of the present invention are those listed in Table 3 below:

**Table 3 : Beta-2 nAChR NAMs**

| **Molecules** | **Reaxys ID** | **CAS Registry Number** | **Bibliographic references** |
|---|---|---|---|
| Amantadine | 2204333 | 768-94-5 | Lee RH et al. 2012 |
| UCI-30002 | | | Bridges et al. (2008) |
| COB-3 | | | Orac CM, 2012 |
| KAB-18 | | | Gonzalez-Cestari et al. 2009 |
| Lynx-1, -2 | | | Miwa et al. 1999 |
| COB-1 | | | Henderson BJ, 2012 |
| COB-2 | | | Henderson BJ, 2012 |
| DDR-15 | | | Henderson BJ, 2012 |
| DDR-13 | | | Henderson BJ, 2012 |
| DDR-18 | | | Henderson BJ, 2012 |
| APB-8 | | | Henderson BJ, 2012 |
| DDR-1 | | | Henderson BJ, 2012 |
| DDR-2 | | | Henderson BJ, 2012 |
| KAB-21 | | | Brandon et al. (2010) |
| DDR-19 | | | Brandon et al. (2010) |
| KAB-24 | | | Brandon et al. (2010) |
| KAB-22 | | | Brandon et al. (2010) |
| KAB-23 | | | Brandon et al. (2010) |
| KAB-19 | | | Brandon et al. (2010) |
| KAB-20 | | | Brandon et al. (2010) |
| KAB-30 | | | Brandon et al. (2010) |
| IB-2 | | | Brandon et al. (2010) |
| PPB-12 | | | Brandon et al. (2010) |
| PPB-13 | | | Brandon et al. (2010) |
| IB-4 | | | Brandon et al. (2010) |
| COB-4 | | | Brandon et al. (2010) |
| LYPD6 | | | Darvas et al. (2009) |
| LYPD6B | | | Ochoa et al. (2016) |
| PSCA | | | Jensen MM, 2015 |
| PATE-M | | | Levitin et al. 2008 |
| SLURP1, SLURP2 | | | Fujii et al. 2014 |
| ly6A/E | | | Wu et al., 2015 |
| ly6B-G | | | Wu et al., 2015 |
| Ly6g6e | | | Wu et al., 2015 |
| ly6H | | | Pudifoot et al 2015 |
| SLEEPLESS | | | Wu et al.. 2014 |
| Odr-2 | | | Gottschalk A , 2006 |
| Boudin | | | Hijazi et al. (2009) |
| Nl-lynxl | | | Liu Z, 2009 |
| Nl-lynx2 | | | Liu Z, 2009 |
| Prlynxl | | | Choo YM, 2008 |
| Erabutoxin b | 15363800 | | Low et al., 1976 |
| Bucandin | | | Torres et al (2001) |
| Candoxin | 8971936 | | Nirthanan et al., 2002 |
| Fasciculins | | | Nirthanan & Gwee, (2004) |
| alpha-colubritoxin | | | Fry et al. 2003 Saviola et al. 2014 |

Alpha-7 nAchR NAMs are known in the art. They are for example EVP-6124, whose CAS number is 550999-74-1. This molecule is an alpha-7 NAM when used at high concentrations (Veys K, 2015). Another alpha-7 negative modulator is S 76892, developed by Servier (Danober L et al, Biochemical Pharmacology, abstract of 2015).

Preferred alpha-7 nAChR NAMs that can be used in the context of the present invention are those listed in Table 4 below:

**Table 4 : Alpha-7 nAChR NAMs**

| **Molecules** | **Reaxys Registry Number** | **Number** | **CAS Registry Bibliographic references** |
|---|---|---|---|
| 1,2,3,3a,4,8b-hexahydro-2-benzyl-6-N,N-dimethylamino-1-methylindeno[1,2,-b]pyrrole (HDMP) | 21876411 | | Galya R. 2010 |
| 2,3,6MP-TQS, cistrans-4-(2,3,6-trimethylphenyl)-3a,4,5,9b-tetrahydro-3H-cyclopenta[c]quinoline-8-sulfonamide | | | JasKiran K. 2015 |
| 2,6MP-TQS, cis-trans-4-(2,6-dimethylphenyl)-3a,4,5,9b-tetrahydro-3H cyclopenta[c] quino line-8- sulfonamide | | | JasKiran K. 2015 |
| BNC210 | | | S.M. O'Connor, 2014 |
| EVP-6124 | | 550999-74-1 | Veys K 2015 |
| Methanandamide | | | Baranowska U (2008) |

Identification and/or characterization of additional beta-2 and alpha-7 nAChR NAMs may be accomplished using *in vitro* and/or *in vivo* assays known in the art.

In the context of the present invention, it is particularly preferred to use antagonists or NAMs that block both alpha-7 containing nAChR and beta-2 containing nAChR.

Preferred molecules that can be used in this aim are those listed in Table 5 below:

**Table 5 : Alpha-7 and beta-2 nAChRs antagonists / NAMs**

| **Molecule** | **Reaxys Registry Number** | **CAS Registry Number** | **Bibliographic references** |
|---|---|---|---|
| Anandamide | 7079463 | 94421-68-8 | Spivak CE et al, 2007 Oz M. et al, 2003 |
| α-Conotoxin (α-ctx) | 14749937 | 175735-93-0 | Whiteaker P, et al. (2007) Janes, 2005 Cartier et al (1996) |
| Memantine | 2075983 | 19982-08-2 | Aracava Y, 2005 |
| SLURP-1 | | | Lyukmanova EN, 2016 |

### E. Therapeutic Compositions

In some embodiments of the methods, the subject is provided not only with beta-2 nAChRs antagonist(s) and/or modulator(s), but also with alpha-7 nAChRs antagonist(s) and/or alpha-7 nAChRs modulator(s). Therefore, the therapeutic compositions of the invention contain either beta-2 nAChRs antagonist(s) and/or modulator(s) alone, or beta-2 nAChRs antagonist(s) and/or modulator(s) along with alpha-7 nAChRs antagonist(s) and/or modulator(s) (as defined in Tables 1-5 above).

The beta-2 nAChR antagonist and/or beta-2 nAChR NAM (optionally along with alpha-7 nAChR antagonist and/or alpha-7 nAChR NAM) is typically formulated into a suitable pharmaceutical preparation for administration to a subject. Suitable examples include solutions, suspensions, tablets, dispersible tablets, pills, capsules, powders, sustained release formulations or elixirs, for oral administration or in sterile solutions or suspensions for parenteral administration, as well as transdermal patch preparation and dry powder inhalers. Typically, the nAChR antagonist and/or NAM is formulated into pharmaceutical compositions using techniques and procedures well known in the art (see, e.g., Ansel Introduction to Pharmaceutical Dosage Forms, Fourth Edition 1985, 126).

In the compositions, effective concentrations of nAChR antagonist and/or NAM, or pharmaceutically acceptable derivatives, is (are) mixed with a suitable pharmaceutical carrier or vehicle.

Pharmaceutically acceptable derivatives include acids, bases, enol ethers and esters, salts, esters, hydrates, solvates and prodrug forms. The derivative is selected such that its pharmacokinetic properties are superior with respect to at least one characteristic to the corresponding neutral agent. The nAChR antagonist and/or NAM may be derivatized prior to formulation.

The concentrations of the nAChR antagonist and/or NAM in the compositions are effective for delivery of an amount, upon administration, that treats one or more of the symptoms of at least one disease state.

Typically, by way of example and without limitation, the compositions are formulated for single dosage administration. To formulate a composition, the weight fraction of nAChR antagonist and/or NAM is dissolved, suspended, dispersed or otherwise mixed in a selected vehicle at an effective concentration such that the treated condition is relieved or ameliorated. Pharmaceutical carriers or vehicles suitable for administration of the nAChR antagonist and/or NAM include any such carriers known to those skilled in the art to be suitable for the particular mode of administration.

In addition, the nAChR antagonist and/or NAM may be formulated as the sole active agent in the composition or may be combined with other active agents. Liposomal suspensions, including tissue-targeted liposomes, may also be suitable as pharmaceutically acceptable carriers. These may be prepared according to methods known to those skilled in the art. For example, liposome formulations may be prepared as described in U.S. Patent No. 4,522,811. Briefly, liposomes such as multilamellar vesicles (MLV's) may be formed by drying down egg phosphatidyl choline and brain phosphatidyl serine (7:3 molar ratio) on the inside of a flask. A solution of a nAChR antagonist and/or NAM provided herein in phosphate buffered saline lacking divalent cations (PBS) is added and the flask shaken until the lipid film is dispersed. The resulting vesicles are washed to remove unencapsulated nAChR antagonist and/or NAM, pelleted by centrifugation, and then resuspended in PBS.

The active nAChR antagonist and/or NAM is included in the pharmaceutically acceptable carrier in an amount sufficient to exert a therapeutically useful effect in the absence of undesirable side effects on the subject. The therapeutically effective concentration may be determined empirically by testing the agents in in vitro and in vivo systems described herein or known in the art and then extrapolated from there for dosages for humans.

The concentration of active nAChR antagonist and/or NAM in the pharmaceutical composition will depend on absorption, inactivation and excretion rates of the nAChR antagonist and/or NAM, the physicochemical characteristics of said agent, the dosage schedule, and amount administered as well as other factors known to those of skill in the art. For example, the amount that is delivered is sufficient to treat at least one disease state.

Typically a therapeutically effective dosage should produce a serum concentration of nAChR antagonist and/or NAM of from about 0.1 ng/ml to about 50-100 µg/ml, for example. The pharmaceutical compositions typically should provide a dosage of from about 0.001 mg to about 2000 mg of nAChR antagonist and/or NAM per kilogram of body weight per day, such as from about 0.01 mg to about 200 mg of nAChR antagonist and/or NAM per kilogram of body weight per day, or from about 0.1 mg to about 20 mg of nAChR antagonist and/or NAM per kilogram of body weight per day, or from about 1 mg to about 10 mg of nAChR antagonist and/or NAM per kilogram of body weight per day, or from about 1 mg to about 5 mg of nAChR antagonist and/or NAM per kilogram of body weight per day. Pharmaceutical dosage unit forms are prepared to provide from about 1 mg to about 1000 mg, such as from about 10 to about 500 mg of said active agent or a combination of said agents per dosage unit form.

The nAChR antagonist and/or NAM may be administered at once, or may be divided into a number of smaller doses to be administered at intervals of time. It is understood that the precise dosage and duration of treatment is a function of the disease state being treated and may be determined empirically using known testing protocols or by extrapolation from in vivo or in vitro test data. It is to be noted that concentrations and dosage values may also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed methods.

Thus, effective concentrations or amounts of one or more nAChR antagonist and/or NAM or pharmaceutically acceptable derivatives thereof are mixed with a suitable pharmaceutical carrier or vehicle for systemic, topical or local administration to form pharmaceutical compositions. A nAChR antagonist and/or NAM is included in an amount effective for treating at least one disease state. The concentration of said active agent in the composition will depend on absorption, inactivation, excretion rates of said active agent, the dosage schedule, amount administered, particular formulation as well as other factors known to those of skill in the art.

The compositions are intended to be administered by a suitable route, including by way of example and without limitation orally, parenterally, rectally, topically and locally. For oral administration, capsules and tablets can be used. The compositions are in liquid, semi-liquid or solid form and are formulated in a manner suitable for each route of administration.

Solutions or suspensions used for parenteral, intradermal, subcutaneous, or topical application can include any of the following components, in any combination: a sterile diluent, including by way of example without limitation, water for injection, saline solution, fixed oil, polyethylene glycol, glycerine, propylene glycol or other synthetic solvent; antimicrobial agents, such as benzyl alcohol and methyl parabens; antioxidants, such as ascorbic acid and sodium bisulfite; chelating agents, such as ethylenediaminetetraacetic acid (EDTA); buffers, such as acetates, citrates and phosphates; and agents for the adjustment of tonicity such as sodium chloride or dextrose. Parenteral preparations can be enclosed in ampoules, disposable syringes or single or multiple dose vials made of glass, plastic or other suitable material.

In instances in which the nAChR antagonist and/or NAM exhibit insufficient solubility, methods for solubilizing said agents may be used. Such methods are known to those of skill in this art, and include, but are not limited to, using co-solvents, such as dimethylsulfoxide (DMSO), using surfactants, such as TWEEN^{®}, or dissolution in aqueous sodium bicarbonate. Pharmaceutically acceptable derivatives of said agents may also be used in formulating effective pharmaceutical compositions.

Upon mixing or addition of the nAChR antagonist and/or NAM, the resulting mixture may be a solution, suspension, emulsion or the like. The form of the resulting mixture depends upon a number of factors, including the intended mode of administration and the solubility of the agent in the selected carrier or vehicle. The effective concentration is sufficient for treating one or more symptoms of at least one disease state.

The pharmaceutical compositions are provided for administration to humans and animals in unit dosage forms, such as tablets, capsules, pills, powders, granules, sterile parenteral solutions or suspensions, and oral solutions or suspensions, and oil-water emulsions containing suitable quantities of nAChR antagonist and/or NAM or pharmaceutically acceptable derivatives thereof. The pharmaceutically therapeutically nAChR antagonist and/or NAM and derivatives thereof are typically formulated and administered in unit-dosage forms or multiple-dosage forms. Unit-dose foams as used herein refers to physically discrete units suitable for human and animal subjects and packaged individually as is known in the art. Each unit-dose contains a predetermined quantity of the therapeutically nAChR antagonist and/or NAM sufficient to produce the desired therapeutic effect, in association with the required pharmaceutical carrier, vehicle or diluent. Examples of unit-dose forms include ampoules and syringes and individually packaged tablets or capsules. Unit-dose forms may be administered in fractions or multiples thereof. A multiple-dose form is a plurality of identical unit-dosage forms packaged in a single container to be administered in segregated unit-dose form. Examples of multiple-dose forms include vials, bottles of tablets or capsules or bottles of pints or gallons. Hence, multiple dose form is a multiple of unit-doses which are not segregated in packaging.

The composition can contain along with the nAChR antagonist and/or NAM, for example and without limitation: a diluent such as lactose, sucrose, dicalcium phosphate, or carboxymethylcellulose; a lubricant, such as magnesium stearate, calcium stearate and talc; and a binder such as starch, natural gums, such as gum acacia gelatin, glucose, molasses, polyvinylpyrrolidone, celluloses and derivatives thereof, povidone, crospovidones and other such binders known to those of skill in the art. Liquid pharmaceutically administrable compositions can, for example, be prepared by dissolving, dispersing, or otherwise mixing an nAChR antagonist and/or NAM as defined above and optional pharmaceutical adjuvants in a carrier, such as, by way of example and without limitation, water, saline, aqueous dextrose, glycerol, glycols, ethanol, and the like, to thereby form a solution or suspension. If desired, the pharmaceutical composition to be administered may also contain minor amounts of nontoxic auxiliary substances such as wetting agents, emulsifying agents, or solubilizing agents, pH buffering agents and the like, such as, by way of example and without limitation, acetate, sodium citrate, cyclodextrin derivatives, sorbitan monolaurate, triethanolamine sodium acetate, triethanolamine oleate, and other such agents. Actual methods of preparing such dosage forms are known, or will be apparent, to those skilled in this art; for example, see Remington's Pharmaceutical Sciences, Mack Publishing Company, Easton, Pa., 15th Edition, 1975. The composition or formulation to be administered will, in any event, contain a quantity of the nAChR antagonist and/or NAM in an amount sufficient to alleviate the symptoms of the treated subject.

Dosage forms or compositions containing the nAChR antagonist and/or NAM in the range of 0.005% to 100% with the balance made up from non-toxic carrier may be prepared. For oral administration, a pharmaceutically acceptable non-toxic composition is formed by the incorporation of any of the normally employed excipients, such as, for example and without limitation, pharmaceutical grades of mannitol, lactose, starch, magnesium stearate, talcum, cellulose derivatives, sodium crosscarmellose, glucose, sucrose, magnesium carbonate or sodium saccharin. Such compositions include solutions, suspensions, tablets, capsules, powders and sustained release formulations, such as, but not limited to, implants and microencapsulated delivery systems, and biodegradable, biocompatible polymers, such as collagen, ethylene vinyl acetate, polyanhydrides, polyglycolic acid, polyorthoesters, polylactic acid and others. Methods for preparation of these compositions are known to those skilled in the art. The contemplated compositions may contain 0.001%-100% of said nAChR antagonist and/or NAM, such as 0.1-85%, or such as 75-95%. [0186] The nAChR antagonist and/or NAM or pharmaceutically acceptable derivatives may be prepared with carriers that protect said agent against rapid elimination from the body, such as time release formulations or coatings. The compositions may include other active agents to obtain desired combinations of properties. nAChR antagonist and/or NAM or pharmaceutically acceptable derivatives thereof, may also be advantageously administered for therapeutic or prophylactic purposes together with another pharmacological agent known in the general art to be of value in treating at least one disease state.

Oral pharmaceutical dosage forms include, by way of example and without limitation, solid, gel and liquid. Solid dosage forms include tablets, capsules, granules, and bulk powders. Oral tablets include compressed, chewable lozenges and tablets which may be enteric-coated, sugar-coated or film-coated. Capsules may be hard or soft gelatin capsules, while granules and powders may be provided in non-effervescent or effervescent forms with the combination of other ingredients known to those skilled in the art.

In some embodiments, the formulations are solid dosage forms, such as capsules or tablets. The tablets, pills, capsules, troches and the like can contain any of the following ingredients, or agents of a similar nature: a binder; a diluent; a disintegrating agent; a lubricant; a glidant; a sweetening agent; and a flavoring agent.

Examples of binders include, by way of example and without limitation, microcrystalline cellulose, gum tragacanth, glucose solution, acacia mucilage, gelatin solution, sucrose, and starch paste. Lubricants include, by way of example and without limitation, talc, starch, magnesium or calcium stearate, lycopodium and stearic acid. Diluents include, by way of example and without limitation, lactose, sucrose, starch, kaolin, salt, mannitol, and dicalcium phosphate. Glidants include, by way of example and without limitation, colloidal silicon dioxide. Disintegrating agents include, by way of example and without limitation, crosscarmellose sodium, sodium starch glycolate, alginic acid, corn starch, potato starch, bentonite, methylcellulose, agar and carboxymethylcellulose. Coloring agents include, by way of example and without limitation, any of the approved certified water soluble Fl) and C dyes, mixtures thereof; and water insoluble ID and C dyes suspended on alumina hydrate. Sweetening agents include, by way of example and without limitation, sucrose, lactose, mannitol and artificial sweetening agents such as saccharin, and any number of spray dried flavors. Flavoring agents include, by way of example and without limitation, natural flavors extracted from plants such as fruits and synthetic blends of agents which produce a pleasant sensation, such as, but not limited to peppermint and methyl salicylate. Wetting agents include, by way of example and without limitation, propylene glycol monostearate, sorbitan monooleate, diethylene glycol monolaurate, and polyoxyethylene laural ether. Emetic-coatings include, by way of example and without limitation, fatty acids, fats, waxes, shellac, ammoniated shellac and cellulose acetate phthalates. Film coatings include, by way of example and without limitation, hydroxyethylcellulose, sodium carboxymethylcellulose, polyethylene glycol 4000 and cellulose acetate phthalate.

If oral administration is desired, the nAChR antagonist and/or NAM could be provided in a composition that protects it from the acidic environment of the stomach. For example, the composition can be formulated in an enteric coating that maintains its integrity in the stomach and releases the active agent in the intestine. The composition may also be formulated in combination with an antacid or other such ingredient.

When the dosage unit form is a capsule, it can contain, in addition to material of the above type, a liquid carrier such as a fatty oil. In addition, dosage unit forms can contain various other materials which modify the physical form of the dosage unit, for example, coatings of sugar and other enteric agents. The nAChR antagonist and/or NAM can also be administered as a component of an elixir, suspension, syrup, wafer, sprinkle, chewing gum or the like. A syrup may contain, in addition to the nAChR antagonist and/or NAM, sucrose as a sweetening agent and certain preservatives, dyes and colorings and flavors.

The nAChR antagonist and/or NAM can also be mixed with other active materials which do not impair the desired action, or with materials that supplement the desired action, such as antacids, H2 blockers, and diuretics.

Pharmaceutically acceptable carriers included in tablets are binders, lubricants, diluents, disintegrating agents, coloring agents, flavoring agents, and wetting agents. Enteric-coated tablets, because of the enteric-coating, resist the action of stomach acid and dissolve or disintegrate in the neutral or alkaline intestines. Sugar-coated tablets are compressed tablets to which different layers of pharmaceutically acceptable substances are applied. Film-coated tablets are compressed tablets which have been coated with a polymer or other suitable coating. Multiple compressed tablets are compressed tablets made by more than one compression cycle utilizing the pharmaceutically acceptable substances previously mentioned. Coloring agents may also be used in the above dosage forms. Flavoring and sweetening agents are used in compressed tablets, sugar-coated, multiple compressed and chewable tablets. Flavoring and sweetening agents are useful in the formation of chewable tablets and lozenges.

Liquid oral dosage forms include aqueous solutions, emulsions, suspensions, solutions and/or suspensions reconstituted from non-effervescent granules and effervescent preparations reconstituted from effervescent granules. Aqueous solutions include, for example, elixirs and syrups. Emulsions are either oil-in-water or water-in-oil.

Elixirs are clear, sweetened, hydroalcoholic preparations. Pharmaceutically acceptable carriers used in elixirs include solvents. Syrups are concentrated aqueous solutions of a sugar, for example, sucrose, and may contain a preservative. An emulsion is a two-phase system in which one liquid is dispersed in the form of small globules throughout another liquid. Pharmaceutically acceptable carriers used in emulsions are non-aqueous liquids, emulsifying agents and preservatives. Suspensions use pharmaceutically acceptable suspending agents and preservatives. Pharmaceutically acceptable substances used in non-effervescent granules, to be reconstituted into a liquid oral dosage form, include diluents, sweeteners and wetting agents. Pharmaceutically acceptable substances used in effervescent granules, to be reconstituted into a liquid oral dosage form, include organic acids and a source of carbon dioxide. Coloring and flavoring agents may be used in any of the above dosage forms.

Solvents include by way of example and without limitation, glycerin, sorbitol, ethyl alcohol and syrup. Examples of preservatives include without limitation glycerin, methyl and propylparaben, benzoic add, sodium benzoate and alcohol. Non-aqueous liquids utilized in emulsions, include by way of example and without limitation, mineral oil and cottonseed oil. Emulsifying agents, include by way of example and without limitation, gelatin, acacia, tragacanth, bentonite, and surfactants such as polyoxyethylene sorbitan monooleate. Suspending agents include, by way of example and without limitation, sodium carboxymethylcellulose, pectin, tragacanth, Veegum and acacia. Diluents include, by way of example and without limitation, lactose and sucrose. Sweetening agents include, by way of example and without limitation, sucrose, syrups, glycerin and artificial sweetening agents such as saccharin. Wetting agents, include by way of example and without limitation, propylene glycol monostearate, sorbitan monooleate, diethylene glycol monolaurate, and polyoxyethylene lauryl ether. Organic acids include, by way of example and without limitation, citric and tartaric acid. Sources of carbon dioxide include, by way of example and without limitation, sodium bicarbonate and sodium carbonate. Coloring agents include, by way of example and without limitation, any of the approved certified water soluble FD and C dyes, and mixtures thereof. Flavoring agents include, by way of example and without limitation, natural flavors extracted from plants such fruits, and synthetic blends of agents which produce a pleasant taste sensation.

For a solid dosage form, the solution or suspension, in for example propylene carbonate, vegetable oils or triglycerides, is encapsulated in a gelatin capsule. Such solutions, and the preparation and encapsulation thereof, are disclosed in U.S. Patent Nos. 4,328,245; 4,409,239; and 4,410,545. For a liquid dosage form, the solution, for example in a polyethylene glycol, may be diluted with a sufficient quantity of a pharmaceutically acceptable liquid carrier, e.g., water, to be easily measured for administration.

Alternatively, liquid or semi-solid oral formulations may be prepared by dissolving or dispersing the active agent or salt in vegetable oils, glycols, triglycerides, propylene glycol esters (e.g., propylene carbonate) and other such carriers, and encapsulating these solutions or suspensions in hard or soft gelatin capsule shells. Other useful formulations include those set forth in U.S. Patent Nos. Re 28,819 and 4,358,603. Briefly, such formulations include, but are not limited to, those containing an agent provided herein, a dialkylated mono- or poly-alkylene glycol, including, but not limited to, 1,2-dimethoxymethane, diglyme, triglyme, tetraglyme, polyethylene glycol-350-dimethyl ether, polyethylene glycol-550-dimethyl ether, polyethylene glycol-750-dimethyl ether wherein 350, 550 and 750 refer to the approximate average molecular weight of the polyethylene glycol, and one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, thiodipropionic acid and its esters, and dithiocarbamates.

Other formulations include, but are not limited to, aqueous alcoholic solutions including a pharmaceutically acceptable acetal. Alcohols used in these formulations are any pharmaceutically acceptable water-miscible solvents having one or more hydroxyl groups, including, but not limited to, propylene glycol and ethanol. Acetals include, but are not limited to, di(lower alkyl) acetals of lower alkyl aldehydes such as acetaldehyde diethyl acetal.

Tablets and capsules formulations may be coated as known by those of skill in the art in order to modify or sustain dissolution of the nAChR antagonist and/or NAM. Thus, for example and without limitation, they may be coated with a conventional enterically digestible coating, such as phenylsalicylate, waxes and cellulose acetate phthalate.

Parenteral administration, generally characterized by injection, either subcutaneously, intramuscularly or intravenously is also contemplated herein. Injectables can be prepared in conventional forms, either as liquid solutions or suspensions, solid forms suitable for solution or suspension in liquid prior to injection, or as emulsions. Suitable excipients, include by way of example and without limitation, water, saline, dextrose, glycerol or ethanol. In addition, if desired, the pharmaceutical compositions to be administered may also contain minor amounts of non-toxic auxiliary substances such as wetting or emulsifying agents, pH buffering agents, stabilizers, solubility enhancers, and other such agents, such as for example, sodium acetate, sorbitan monolaurate, triethanolamine oleate and cyclodextrins.

Implantation of a slow-release or sustained-release system, such that a constant level of dosage is maintained (see, e.g., U.S. Patent No. 3,710,795) is also contemplated herein. Briefly, a nAChR antagonist and/or NAM is dispersed in a solid inner matrix, e.g., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross-linked partially hydrolyzed polyvinyl acetate, that is surrounded by an outer polymeric membrane, e.g., polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The nAChR antagonist and/or NAM diffuses through the outer polymeric membrane in a release rate controlling step. The percentage of nAChR antagonist and/or NAM contained in such parenteral compositions is highly dependent on the specific nature thereof, as well as the activity of the nAChR antagonist and/or NAM and the needs of the subject.

Parenteral administration of the nAChR antagonist and/or NAM includes intravenous, subcutaneous and intramuscular administrations. Preparations for parenteral administration include sterile solutions ready for injection, sterile dry soluble products, such as lyophilized powders, ready to be combined with a solvent just prior to use, including hypodermic tablets, sterile suspensions ready for injection, sterile dry insoluble products ready to be combined with a vehicle just prior to use and sterile emulsions. The solutions may be either aqueous or nonaqueous.

If administered intravenously, suitable carriers include physiological saline or phosphate buffered saline (PBS), and solutions containing thickening and solubilizing agents, such as glucose, polyethylene glycol, and polypropylene glycol and mixtures thereof.

Pharmaceutically acceptable carriers used in parenteral preparations include aqueous vehicles, nonaqueous vehicles, antimicrobial agents, isotonic agents, buffers, antioxidants, local anesthetics, suspending and dispersing agents, emulsifying agents, sequestering or chelating agents and other pharmaceutically acceptable substances.

Aqueous vehicles include, by way of example and without limitation, Sodium Chloride Injection, Ringers Injection, Isotonic Dextrose Injection, Sterile Water Injection, Dextrose and Lactated Ringers Injection. Nonaqueous parenteral vehicles include, by way of example and without limitation, fixed oils of vegetable origin, cottonseed oil, corn oil, sesame oil and peanut oil. Antimicrobial agents in bacteriostatic or fungistatic concentrations must be added to parenteral preparations packaged in multiple-dose containers which include phenols or cresols, mercurials, benzyl alcohol, chlorobutanol, methyl and propyl p-hydroxybenzoic acid esters, thimerosal, benzalkonium chloride and benzethonium chloride. Isotonic agents include, by way of example and without limitation, sodium chloride and dextrose. Buffers include phosphate and citrate. Antioxidants include sodium bisulfate. Local anesthetics include procaine hydrochloride. Suspending and dispersing agents include sodium carboxymethylcelluose, hydroxypropyl methylcellulose and polyvinylpyrrolidone. Emulsifying agents include Polysorbate 80 (TWEEN^{®} 80). A sequestering or chelating agent of metal ions include EDTA. Pharmaceutical carriers also include, by way of example and without limitation, ethyl alcohol, polyethylene glycol and propylene glycol for water miscible vehicles and sodium hydroxide, hydrochloric acid, citric acid or lactic acid for pH adjustment.

The concentration of the pharmaceutically active nAChR antagonist and/or NAM is adjusted so that an injection provides an effective amount to produce the desired pharmacological effect. The exact dose depends on the age, weight and condition of the patient or animal as is known in the art.

The unit-dose parenteral preparations are packaged in an ampoule, a vial or a syringe with a needle. Preparations for parenteral administration should be sterile, as is known and practiced in the art.

Illustratively, intravenous or intra-arterial infusion of a sterile aqueous solution containing an nAChR antagonist and/or NAM is an effective mode of administration. Another embodiment is a sterile aqueous or oily solution or suspension containing an active agent injected as necessary to produce the desired pharmacological effect.

Injectables are designed for local and systemic administration. Typically a therapeutically effective dosage is formulated to contain a concentration of at least about 0.1% w/w up to about 90% w/w or more, such as more than 1% w/w of the nAChR antagonist and/or NAM to the treated tissue(s). The nAChR antagonist and/or NAM may be administered at once, or may be divided into a number of smaller doses to be administered at intervals of time. It is understood that the precise dosage and duration of treatment is a function of the tissue being treated and may be determined empirically using known testing protocols or by extrapolation from in vivo or in vitro test data. It is to be noted that concentrations and dosage values may also vary with the age of the individual treated. It is to be further understood that for any particular subject, specific dosage regimens should be adjusted over time according to the individual need and the professional judgment of the person administering or supervising the administration of the formulations, and that the concentration ranges set forth herein are exemplary only and are not intended to limit the scope or practice of the claimed formulations.

The nAChR antagonist and/or NAM may be suspended in micronized or other suitable form or may be derivatized, e.g., to produce a more soluble active product or to produce a prodrug or other pharmaceutically acceptable derivative. The form of the resulting mixture depends upon a number of factors, including the intended mode of administration and the solubility of the nAChR antagonist and/or NAM in the selected carrier or vehicle. The effective concentration is sufficient for ameliorating the symptoms of the condition and may be empirically determined.

Lyophilized powders can be reconstituted for administration as solutions, emulsions, and other mixtures or formulated as solids or gels.

The sterile, lyophilized powder is prepared by dissolving an agent provided herein, or a pharmaceutically acceptable derivative thereof, in a suitable solvent. The solvent may contain an excipient which improves the stability or other pharmacological component of the powder or reconstituted solution, prepared from the powder. Excipients that may be used include, but are not limited to, dextrose, sorbital, fructose, corn syrup, xylitol, glycerin, glucose, sucrose or other suitable agent. The solvent may also contain a buffer, such as citrate, sodium or potassium phosphate or other such buffer known to those of skill in the art at, typically, about neutral pH. Subsequent sterile filtration of the solution followed by lyophilization under standard conditions known to those of skill in the art provides the desired formulation. Generally, the resulting solution will be apportioned into vials for lyophilization. Each vial will contain, by way of example and without limitation, a single dosage (10-1000 mg, such as 100-500 mg) or multiple dosages of the agent. The lyophilized powder can be stored under appropriate conditions, such as at about 4°C to room temperature.

Reconstitution of this lyophilized powder with water for injection provides a formulation for use in parenteral administration. For reconstitution, about 1-50 mg, such as about 5-35 mg, for example, about 9-30 mg of lyophilized powder, is added per mL of sterile water or other suitable carrier. The precise amount depends upon the selected nAChR antagonist and/or NAM. Such amount can be empirically determined.

Topical mixtures are prepared as described for the local and systemic administration. The resulting mixture may be a solution, suspension, emulsions or the like and are formulated as creams, gels, ointments, emulsions, solutions, elixirs, lotions, suspensions, tinctures, pastes, foams, aerosols, irrigations, sprays, suppositories, bandages, dermal patches or any other formulations suitable for topical administration.

The nAChR antagonist and/or NAM or pharmaceutically acceptable derivatives thereof may be formulated as aerosols for topical application, such as by inhalation (see, e.g., U.S. Patent Nos. 4,044,126, 4,414,209, and 4,364,923, which describe aerosols for delivery of a steroid useful for treatment of inflammatory diseases, particularly asthma). These formulations for administration to the respiratory tract can be in the form of an aerosol or solution for a nebulizer, or as a micro fine powder for insufflation, alone or in combination with an inert carrier such as lactose. In such a case, the particles of the formulation will, by way of example and without limitation, have diameters of less than about 50 microns, such as less than about 10 microns.

The nAChR antagonist and/or NAM may be formulated for local or topical application, such as for topical application to the skin and mucous membranes, such as in the eye, in the form of gels, creams, and lotions and for application to the eye or for intracisternal or intraspinal application. Topical administration is contemplated for transdermal delivery and also for administration to the eyes or mucosa, or for inhalation therapies. Nasal solutions of the nAChR antagonist and/or NAM alone or in combination with other pharmaceutically acceptable excipients can also be administered.

These solutions, particularly those intended for ophthalmic use, may be formulated, by way of example and without limitation, as about 0.01% to about 10% isotonic solutions, pH about 5-7, with appropriate salts.

Other routes of administration, such as transdermal patches, and rectal administration are also contemplated herein.

Transdermal patches, including iotophoretic and electrophoretic devices, are well known to those of skill in the art. For example, such patches are disclosed in U.S. Patent Nos. 6,267,983, 6,261,595, 6,256,533, 6,167,301, 6,024,975, 6,010715, 5,985,317, 5,983,134, 5,948,433, and 5,860,957.

Pharmaceutical dosage forms for rectal administration are rectal suppositories, capsules and tablets for systemic effect. Rectal suppositories are used herein mean solid bodies for insertion into the rectum which melt or soften at body temperature releasing one or more pharmacologically or therapeutically active ingredients. Pharmaceutically acceptable substances utilized in rectal suppositories are bases or vehicles and agents to raise the melting point. Examples of bases include cocoa butter (theobroma oil), glycerin-gelatin, carbowax (polyoxyethylene glycol) and appropriate mixtures of mono-, di- and triglycerides of fatty acids. Combinations of the various bases may be used. Agents to raise the melting point of suppositories include spermaceti and wax. Rectal suppositories may be prepared either by the compressed method or by molding. The typical weight of a rectal suppository is, by way of example and without limitation, about 2 to 3 gm.

Tablets and capsules for rectal administration are manufactured using the same pharmaceutically acceptable substance and by the same methods as for formulations for oral administration.

The nAChR antagonist and/or NAM, or pharmaceutically acceptable derivatives thereof, may also be formulated to be targeted to a particular tissue, receptor, or other area of the body of the subject to be treated. Many such targeting methods are well known to those of skill in the art. Such targeting methods are contemplated herein for use in the instant compositions. For non-limiting examples of targeting methods, see, e.g., U.S. Patent Nos. 6,316,652, 6,274,552, 6,271,359, 6,253,872, 6,139,865, 6,131,570, 6,120,751, 6,071,495, 6,060,082, 6,048,736, 6,039,975, 6,004,534, 5,985,307, 5,972,366, 5,900,252, 5,840,674, 5,759,542 and 5,709,874. In some embodiments, liposomal suspensions, including tissue-targeted liposomes, such as tumor-targeted liposomes, may also be suitable as pharmaceutically acceptable carriers. These may be prepared according to methods known to those skilled in the art. For example, liposome formulations may be prepared as described in U.S. Patent No. 4,522,811. Briefly, liposomes such as multilamellar vesicles (MLV's) may be formed by drying down egg phosphatidyl choline and brain phosphatidyl serine (7:3 molar ratio) on the inside of a flask. A solution of nAChR antagonist and/or NAM provided herein in phosphate buffered saline lacking divalent cations (PBS) is added and the flask shaken until the lipid film is dispersed. The resulting vesicles are washed to remove unencapsulated agent, pelleted by centrifugation, and then resuspended in PBS.

### F. Methods of Reducing Development of Mild Cognitive Impairment and/or Learning Disorders and/or Memory Disorders in a Subject Suspected to Have or Suffering from Alzheimer's Disease (AD)

The invention provides methods of reducing development of mild cognitive impairment and/or learning disorders and/or memory disorders in a subject suspected to have or suffering from AD. In some embodiments, the subject has been diagnosed with mild cognitive impairment. In some embodiments, it has been determined that the subject with mild cognitive impairment is suspected to have AD, namely a subject without a disease's clear diagnostic. In some embodiments, the subject has been diagnosed with AD.

In some embodiments, the methods comprise administering a therapeutically effective amount of a beta-2 (and optionally alpha-7) nAChR antagonist and/or beta-2 (and optionally alpha-7) nAChR NAM to the subject over a therapeutic dosing period. The therapeutically effective amount is an amount that ameliorates, delays, or prevents any of the clinical signs, behaviors or events associated with MCI, learning disorders, memory disorders or AD, over the therapeutic dosing period. Disease progression is assessed using any method known in the art. In some embodiments, disease progression means an increase in severely of at lease one disease symptom. In some embodiments, disease progression means onset of at least one disease symptom that is characteristic of a more severe disease state. A therapeutic dosing period is a period of time over which disease progression normally occurs at a detectable rate in a subject diagnosed with mild cognitive impairment or learning disorders or memory disorders or AD. In some embodiments, the therapeutic dosing period is, for example, selected from one month to two years, one month to eighteen months, one month to twelve months, one month to six months or one month to three months. In some embodiments, the therapeutic dosing period is, for example, selected from three months to two years, three months to eighteen months, three months to twelve months, or three months to six months. In some embodiments, the therapeutic dosing period is, for example, selected from six months to two years, six months to eighteen months, or six months to twelve months. In some embodiments, the therapeutic dosing period is at least one month, at least three months, at least six months, at least nine months, at least twelve months, at least eighteen months, or at least two, five, eight, ten, fifteen, twenty, twenty-five, thirty, thirty-five, forty years.

In some embodiments, the rate of neurodegeneration in the subject is reduced. In some embodiments, the rate of neurodegeneration in the subject's cerebral cortex is reduced. In some embodiments, the rate of neurodegeneration in the subject's hippocampus is reduced. In some embodiments, the rate of neurodegeneration in the subject's hippocampal interneurons is reduced.

In another aspect, the invention provides the use of beta-2 (and optionally alpha-7) nAChR antagonist(s) and/or beta-2 (and optionally alpha-7) nAChR NAM(s) to prepare a medicament intended to prevent AD or treat the AD in a subject in need thereof. More specifically, said medicament is intended to prevent AD or treat or reduce the rate of development of mild cognitive impairment and/or learning disorders and/or memory disorders in a subject suspected to have or suffering from AD.

In another aspect, the invention provides a composition comprising an effective amount of beta-2 (and optionally alpha-7) nAChR antagonist(s) and/or beta-2 (and optionally alpha-7) nAChR NAM(s) for use for preventing AD or treating the AD in a subject in need thereof. More specifically, said composition is for use for preventing AD, treating and/or reducing the rate of development of mild cognitive impairment and/or learning disorders and/or memory disorders in a subject suspected to have or suffering from AD.

In another aspect, the invention provides a kit-of-parts or a combination product comprising :
a) a beta-2 nAChR antagonist and/or beta-2 nAChR NAM;
   and
b) an alpha-7 nAChR antagonist and/or alpha-7 nAChR NAM,
for simultaneous, separated or staggered use for preventing AD or treating a subject suspected to have or suffering from AD.

Also, the invention provides a kit-of-parts or a combination product comprising :
a) a beta-2 nAChR antagonist and/or beta-2 nAChR NAM;
   and
b) an alpha-7 nAChR antagonist and/or alpha-7 nAChR NAM,
for simultaneous, separated or staggered use for reducing the rate of development of mild cognitive impairment and/or learning disorders and/or memory disorders in a subject suspected to have or suffering from AD.

In these aspects, the beta-2 nAChR antagonist and/or beta-2 nAChR NAM reduces acetylcholine signaling through at least one (preferably two) beta-2 -containing nAChR(s).

In preferred embodiments, the beta-2 nAChR antagonist and/or beta-2 nAChR NAM reduces acetylcholine signaling through any nAChR comprising at least one, two or three beta-2 subunits, and preferably two beta-2 subunit(s).

In more preferred embodiments, the beta-2 nAChR antagonist and/or beta-2 nAChR NAM reduces acetylcholine signaling through at least one nAChR selected from the group consisting of: alpha-2 alpha 5 beta-2, alpha-2 beta-2, alpha-3 beta-2, alpha-4 beta-2, alpha-6 beta-2, alpha-7 beta-2, alpha-3 alpha-5 beta-2, alpha-3 alpha-6 beta-2, alpha-4 alpha-5 beta-2, alpha-4 beta-2 beta-3, and alpha-5 alpha-6 beta-2; preferably selected from the group consisting of: alpha-2 alpha-5 beta-2, alpha-4 beta-2, alpha-3 beta-2 and alpha-2 beta-2 ; and more preferably through alpha-2 beta-2.

In preferred embodiments, the beta-2 (and optionally alpha-7) nAChR antagonist and/or beta-2 (and optionally alpha-7) nAChR NAM is administered to the subject at a regular dosing interval of from three to twenty-four hours for the therapeutic dosing period. In some embodiments, the beta-2 nAChR antagonist (and optionally alpha-7) and/or beta-2 (and optionally alpha-7) nAChR NAM is administered to the subject at a regular dosing interval selected from three hours to six hours, four hours to eight hours, or six hours to twelve hours.

In preferred embodiments, a beta-2 nAChR antagonist is administered to the subject. Said beta-2 nAChR antagonist is preferably selected from the group described in Table 1 above.

In preferred embodiments, a beta-2 nAChR NAM is administered to the subject. Said beta-2 nAChR NAM is preferably selected from the group described in Table 3 above.

In preferred embodiments, alpha-7 nAChR antagonist(s) and/or alpha-7 nAChR NAM(s) is also administered to the subject. Exemplary useful antagonists and modulators have been described in Tables 2 and 4 above. They are specifically MLA, EVP-6124 and S 76892.

Particularly preferred compounds to be used in these embodiments are those listed in Table 5, that can block both alpha-7 and beta-2 containing nAChR.

In some embodiments, the methods further comprise administering an inhibitor of acetylcholine esterase to the subject during the therapeutic dosing period. In some embodiments, the inhibitor of acetylcholine esterase is selected from: Tacrine, also known as tetrahydroaminoacridine (THA'); Rivastigmine; Donepezil; Galantamine; Carbamates; Physostigmine; Neostigmine; Pyridostigmine; Ambenonium; Demecarium; Phenanthrene derivatives; Caffeine - noncompetitive (also an Adenosine receptor antagonist); Rosmarinic acid - ester of Caffeic acid found in plants species of Lamiaceae family; Alpha-Pinene - noncompetitive reversible; Piperidines; Edrophonium; Huperzine A; Ladostigil; Ungeremine; Lactucopicrin.

In some embodiments, the methods further comprise administering an NMDA (N-methyl-D-aspartate) receptor antagonist to the subject during the therapeutic dosing period. In some embodiments, the NMDA (N-methyl-D-aspartate) receptor antagonist is Memantine.

In some embodiments, the methods further comprise administering an antibody directed against the Abeta to the subject during the therapeutic dosing period. In some embodiments, the anti-Abeta antibody is a humanized monoclonal anti-Abeta antibody. In somme embodiments, the humanized monoclonal anti-Abeta antibody is selected from the group consisting of: bapineuzumab, solanezumab, gantenerumab, crenezumab, GSK933776 and BAN-2401. In some embodiments, the antibody directed against the Abeta is a human polyclonal anti-Abeta antibody. In some embodiments, the methods further comprise administering an gamma- or beta- secretase inhibitor to the subject during the therapeutic dosing period. In some embodiments, the gamma-secretase inhibitor is selected from the group consisting of : semagacestat, ELND006, avagacestat, begacestat, NIC5-15 and CHF-5074. In some embodiments, the beta-secretase inhibitor is selected from the group consisting of: MK-8931, LY2886721, AZD3293, LY3314814, E2609.

In some embodiments, the methods further comprise administering an agent directed against the tau protein to the subject during the therapeutic dosing period. In some embodiments, the agent directed against the tau protein is selected from the group consisting of : an inhibitor of tau hyperphosphorylation (such as LMTX), epothilone D, TPI-287, an anti-tau vaccine (sucha as AADvac1 or ACI-35), and a GSK-3beta inhibitor (such as Tideglusib, intranasal Humulin R or intranasal Glulizine).

In some embodiments, the methods further comprise administering a serotonin 5-HT₆ receptor antagonist to the subject during the therapeutic dosing period. In some embodiments, the serotonin 5-HT₆ receptor antagonist is selected from the group consisting of : Idalopirdine (LUAE58054) and SB 742457.

In some embodiments, the methods further comprise administering an histamine H₃ receptor antagonist to the subject during the therapeutic dosing period. In some embodiments, the H₃ histamine receptor antagonist is selected from the group consisting of : ABT-288, GSK239512.

In some embodiments, the methods further comprise administering an inhibitor of the advanced glycation endproduct receptor (RAGE) to the subject during the therapeutic dosing period. In some embodiments, the RAGE inhibitor is Azeliragon.

In some embodiments, the methods further comprise administering a partial selective agonist of the alpha-7- nAChR to the subject during the therapeutic dosing period. In some embodiments, the partial selective agonist of the alpha-7- nAChR is Encenicline (EVP-6124 at low concentration, MT-4666).

In some embodiments, the methods further comprise administering an inhibitor of tumor necrosis factor-alpha (TNF-alpha) release to the subject during the therapeutic dosing period. In some embodiments, the inhibitor of TNF-alpha release is Thalidomide.

In some embodiments, the methods further comprise administering a glucagon-like peptide-1 (GLP-1) receptor agonist to the subject during the therapeutic dosing period. In some embodiments, the GLP-1 receptor agonist is Liraglutide.

In some embodiments, the methods further comprise administering an inhibitor of calcium channels to the subject during the therapeutic dosing period. In some embodiments, the inhibitor of calcium channels is Nilvadipine.

In some embodiments, the methods further comprise administering an anti-Abeta vaccine to the subject during the therapeutic dosing period. In some embodiments, the anti-Abeta vaccine is selected from the group consisting of: AN-1792, CAD106 and ACC-001.

### BRIEF DESCRIPTION OF THE DRAWINGS

**Figures 1A to 1D****:** Characterization ofhAPP-SLA. A: Lentivirus construct encoding for the 695 amino acid isoform of the human APP harbouring three pathogenic mutations (hAPP-SLA). The mutations on the APP sequence are highlighted in red. This peptide sequence is also referenced as SEQ ID NO:11. B: Amplification of the hAPP-SLA mRNA showing its expression only in brain extract derived from hAPP-SLA injected mice; M: molecular weight marker; lane 1: brain extract from hAPP-SLA injected mouse; lane 2: brain extract from GFP injected mouse; lane 3: non-injected mouse; lane 4: mRNA negative control (water). C: Quantification of fluorescence intensity of GFP maker in DG, CA1 and CA3 showing that the majority of the fluorescent signal is found in DG. The selected areas for the quantifications are: granular cell layer in DG, pyramidal cell layer in CA1 and CA3 (white ROI in figure 1D). The fluorescence intensity was normalised on ROI area (white line). D: Three slices on the anterior-posterior axis, used for fluorescence intensity quantification (n = 20). Scale bars 200 µm.

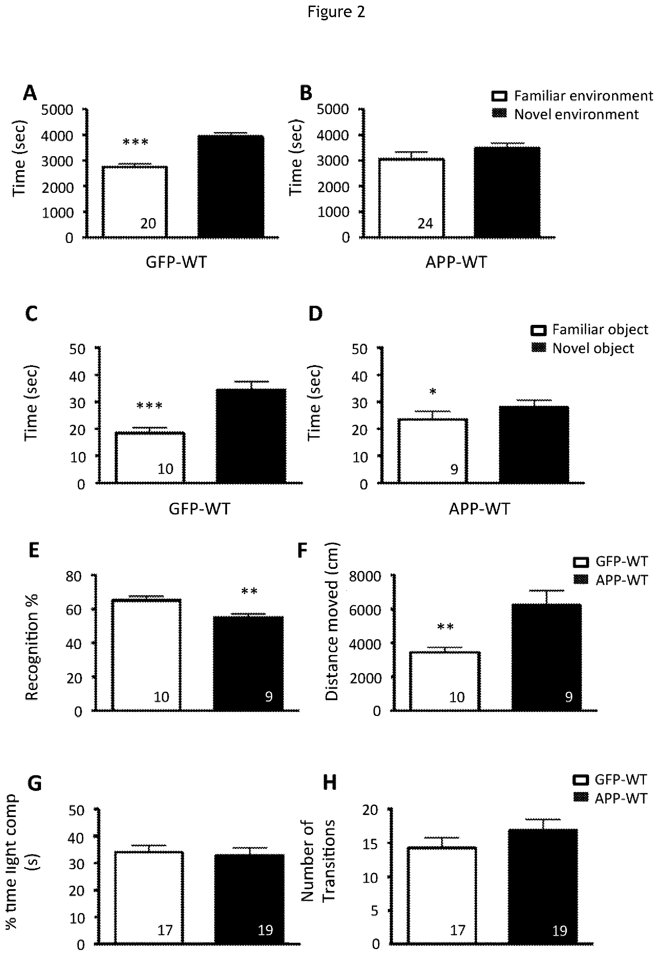

**Figures 2A to 2H****:** hAPP-SLA expression induced memory deficit in WT mice. A-B: NPR task: GFP-WT (2A) spent significantly more time in the novel compartment, while APP-WT (2B) spent an equal amount of time in the familiar and in the novel compartment, indicating a memory deficit (p values: GFP-WT = 0,0003; APP-WT = 0,37). C-D: Memory was also evaluated with the NOR task: GFP-WT spent more time exploring the novel object during the trial phase of the test (2C) (p = 0.0002), APP-WT (2D) as well displayed higher exploration of the novel object (p = 0.047). Recognition percentage index, comparison between GFP-WT and APP-WT. E: GFP-WT mice displayed significantly higher recognition index compared to APP-WT (p = 0.0032), demonstrating the presence of a memory recognition deficit in the latter. F: The quantification of the distance moved in the habituation phase of NOR showed that the APP-WT group was hyperactive compared to GFP-WT (p = 0.0027). G-H: The anxiety level was evaluated with the LDB task. This test showed no difference between the two groups in both the percentage of time spent in lit compartment (2G) (p = 0.7) or in the transitions between compartments (2H) (p = 0.2).

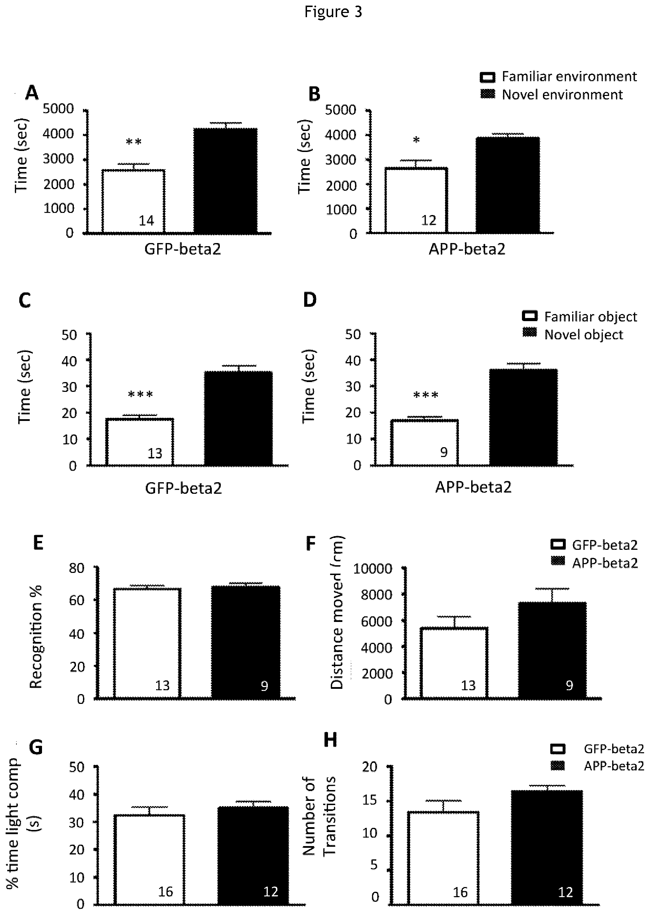

**Figures 3A to 3H****:** Deletion of the beta-2 nAChR subunit prevents the memory deficit. A-B: NPR performed on beta-2 KOs. The GFP-beta-2 (2A) spent more time in the exploration of the novel compartment, a well as the APP-beta-2 (6B), showing that beta-2 deletion prevents the memory loss (p values: GFP-beta-2 = 0.003; APP-beta-2 = 0.017). C-E: NOR test confirmed the result obtained with the novel place recognition task. Neither GFP-beta-2 (6C) (p < 0.0001) nor APP-beta-2 (6D) (p = 0.0001) displayed a memory deficit. The analysis of recognition percentage index for GFP-beta-2 and APP-beta-2 (6E) (p = 0.71) confirmed the absence of a cognitive deficit in mice expressing hAPP-SLA. F) Locomotion analysis showed no significant difference between the groups (p = 0.2). G-H: The DLB task showed that anxiety was not affected. The percentage of time spent in the lit compartment (6G) (p = 0.52) and the transitions between compartments (6H) (p = 0.17) were analyzed and did not show any difference between GFP-beta-2 and APP-beta-2.

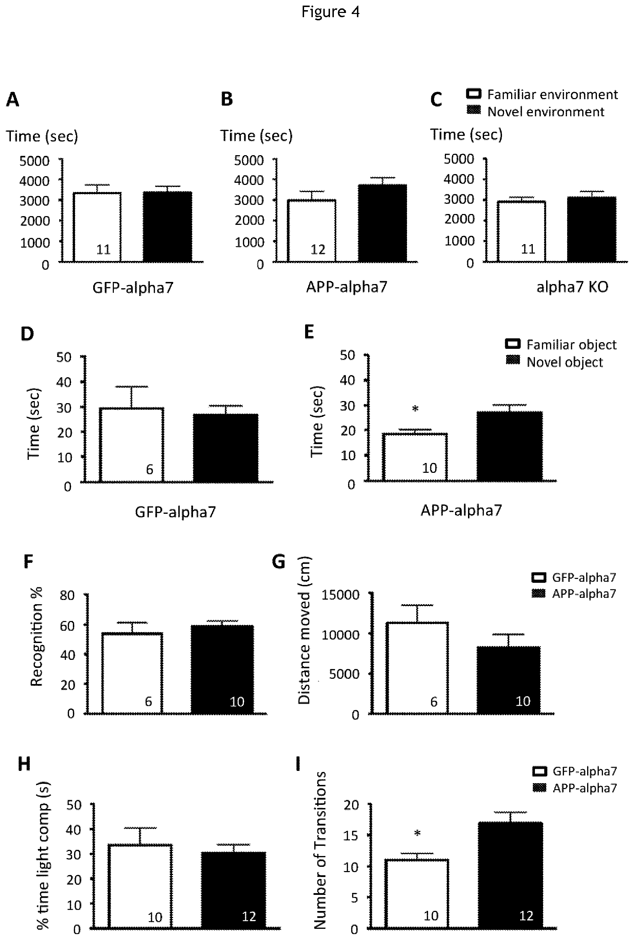

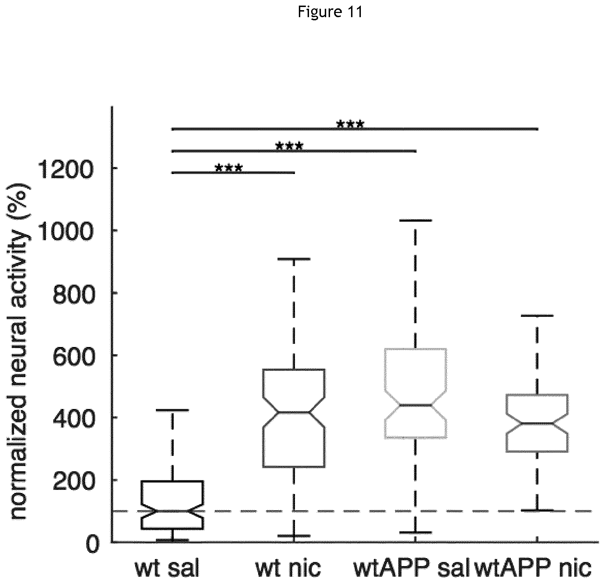

**Figures 4A** **to 41:** The deletion of the alpha-7 nAChR subunit caused a memory deficit independent of the hAPP-SLA expression. A-C: NPR performed on alpha-7 KOs. The GFP-alpha-7 spent the same amount of time in the exploration of the familiar and novel compartment (4A) (p = 0.97) displaying a deficit in recognition memory as well as the APP-alpha-7 (4B) (p = 0.39). We then performed the novel place recognition task on aged-matched non-injected alpha-7 KOs. This group as well displayed memory impairment in this task (4C) (p = 0.66). D-F) NOR test confirmed the memory deficit in GFP-alpha-7 group (D) (p = 0.76), while the APP-alpha-7 group (E) displayed a tendency for higher exploration of the novel object (p = 0.016). However the analysis of recognition percentage did not confirm this result (F) (p = 0.49). G) The analysis of distance moved showed no alteration of locomotion activity (p = 0.29). H-I: No difference in the percentage of time spent in lit the compartment was found between the two groups (4H) (p = 0.66), however, higher number of transitions was detected for the APP-alpha-7 (41) (p = 0.016).

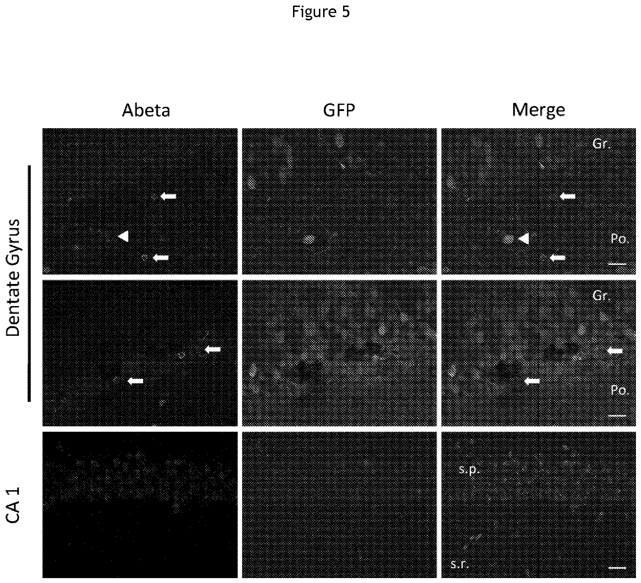

**Figure 5****:** Aβ (red) and GFP (green) immunofluorescence in APP-WT. Aβ immunofluorescence was performed with a VHH specific for the oligomeric form. Aβ oligomers were mainly found in the polymorphic layer of DG (white arrow), in close proximity of virus infected cells (green). Very few GFP positive cells contained Aβ oligomers (arrow head). Aβ oligomers accumulate intracellularly. No Aβ staining was found in the CA1 of hAPP-injected mice, or in GFP-WT (data not shown) (Gr: Granular layer, Po: Polymorphic layer, s.p: stratum pyramidale, s.r: stratum radiatum). Blue: DAPI. Scale bars 20 µm.
**Figure 6****:** Immunofluorescence in beta-2 and alpha-7 KO mice. The immunofluorescence showed that Aβ intracellular staining (red) was found only in DG of APP-beta-2 and APP-alpha-7 groups.
Aβ positive cells were localized in the polymorphic layer of the DG in both beta-2 and alpha-7 KO mice (white arrow). (Gr: Granular layer, Po: Polymorphic layer). Blue: DAPI. Scale bars 20 µm.
**Figure** 7: Immunofluorescence in beta-2 and alpha-7 KO mice. The immunofluorescence showed that Abeta intracellular staining (red) was found only in DG of APP-beta2 and APP-alpha7 groups.
Abeta positive cells were localized in the polymorphic layer of the DG in both beta-2 and alpha-7 KO mice (white arrow). (Gr: Granular layer, Po: Polymorphic layer). Blue: DAPI. Scale bars 20 µm.
**Figure 8****:** Immunofluorescence performed with anti-Ibal antibody. No evident microglia activation was detected comparing WT-GFP (A) and WT-APP (B).
**Figure 9** : Hippocampal electrophysiology after administration of the lentivirus of the invention in the indicated KO mice, as explained in example 9.
**Figure 10****:** Cortical activity in the indicated animals (WT, beta-2 KO, and alpha-7 / beta-2 double KO) after injection of the AAV-hAPP of the invention, as explained in the example 10.
**Figure 11****:** Neural activity in WT mice in which the AAV-hAPP has been injected, along with infusion of alpha-7 and beta-2 nAChR antagonists (nic) or a saline solution (sal), as explained in Example 10.

### EXAMPLES

The following examples serve to more fully describe the manner of using the invention. These examples are presented for illustrative purposes and should not serve to limit the true scope of the invention.

### Example 1: Materials and Methods

*Lentiviral construction.* The lentiviral vector used in this study derived from the third generation systems (Dull et al., 1998). Their principal characteristics are a self-inactivating deletion in the U3 region of the 3'LTR, a chimeric 5'LTR and a woodchuck hepatitis virus post-transcriptional regulatory element sequence (Dull et al., 1998; Zufferey et al., 1999).

LV pTripPGK-hAPP-SLA-FLAG-IRES-eGFP (hAPP-SLA): the hAPP cDNA harbouring the Swedish mutation (KM670/671NL) was recovered from a pcDNA 3.1 plasmid. The cDNA sequence was modified with directed mutagenesis PCR (Agilent Technologies, Stratagene, Santa Clara, CA, USA) to add London (V717I) and Austrian (T714I) mutations (for primers used see Table 6). The same procedure was used to delete the stop codon at APP 3' in order to add a FLAG tag. The obtained construct was inserted into a pGEM-T vector (Promega, Madison, WI, USA), resulting in an APP-SLA-FLAG-pGEM-T plasmid. The hAPP-SLA-FLAG cassette was recovered from pGEM-T vector with XmaI and NheI restriction enzymes and inserted in an LV pTripPGK vector digested with the same enzymes, downstream of the PGK promoter and upstream of the IRES-eGFP sequence. The resulting lentiviral vector was a pTripPGK-hAPP-SLA-FLAG-IRES-eGFP. The lentivirus obtained co-expresses hAPP-SLA and eGFP, the latter was used as reporter gene for the analysis of lentiviral infection diffusion in the selected area.

*Viral production and titration.* The vector plasmid, packaging plasmid (CMVΔ 8.9) and envelope plasmid (CMV-VSVg) were co-transfected in HEK293T cells with Lipofectamine Plus (Life Technologies, Invitrogen, Carlsbad, CA, USA) according to the manufacturer's instructions. After 48h of expression viral particles were collected from cell medium, treated with DNaseI (1:5000; 0.2 µm/ml) and MgCl₂ 2M (1 µl/ml), filtered with 0.45 µm pore filters (Merk Millipore, Darmstadt, Germany), concentrated by ultracentrifugation and stored at - 80°C until use. The titres were determined by quantifying the p24 capsid protein using an HIV-p24 antigen immunoassay (Gentaur Molecular Products, Kampenhout, Belgium) according to the manufacturer's instructions. Before stereotaxic injections, viral stocks were diluted to the concentration of 100 ng/µl of p24 protein with PBS, and a volume of 2 µl was injected.

Experiments were performed with male wild-type mice (C57B1/6J line), and null mutant mice for the alpha-7 (alpha-7 KO mice) or beta-2 (beta-2 KO mice) subunits of the nAChRs. The mutant lines were generated as previously described (Orr-Urtreger et al., 1997; Picciotto et al., 1995). Mice were breed at Charles River Laboratories in isolators under a 12h light-dark cycle. All mice were transported to our facilities at ten weeks of age, housed under a 12h light-dark cycle with ad libitum access to food and water.

*Stereotaxic injections.* 12 week old mice were anesthetized with 1.5% Ketamine (Merial, Lyon, France) plus 0.05% Xylazine (Bayer Healthcare, Lille, France), in PBS. A stereotaxic frame adapted for mice was used. Lentiviruses (2µl at 100ng/µl) were injected bilaterally (flow rate 0.2 µl/min). The following coordinates for the Dentate Gyrus (DG) were used, according to the Paxinos and Franklin Atlas (Paxinos and Franklin, 2004): A/P:-1.8 M/L:±1.2 D/V:-1.9.

*Total RNA extraction and mRNA amplification:* Mice were deeply anesthetized with a lethal dose of Ketamine/Xylazine. Intracardial perfusion with PBS was performed and hippocampi were dissected and collected. The tissue was homogenised with a pestle in Trizol (Life Technologies, Ambion, Carlsbad, CA, USA). Consecutive steps of centrifugation were performed with Chloroform (Sigma-Aldrich, Saint Louis, MO, USA) and Isopropanol (Sigma-Aldrich, Saint Louis, MO, USA). Finally the samples were centrifuged, the pellet recovered, re-suspended in sterile water and stored at -80°C until use. Reverse transcription was performed with the commercial kit Superscript Vilo cDNA synthesis (Life Technologies, Invitrogen, Carlsbad, CA, USA) according to the manufacturer's instructions. Primers used for amplification are listed in Table 6. Primers were designed against the human APP sequence to avoid any amplification of murine APP.

For behavioural experiments, two groups of mice were injected at twelve weeks of age. One group was injected with the hAPP-SLA lentivirus (APP-WT; APP-alpha-7 and APP-beta-2 groups) and the control group was injected with a lentivirus expressing only GFP (GFP-WT; GFP-alpha-7 and GFP- beta-2 groups).

*Novel place recognition (NPR)*. Recognition memory was evaluated in a 'novel place recognition' procedure as a simple behavioral assay of memory that primarily relies on rodent's propensity to explore novelty in the absence of external rules of reinforcement. C57B1/6J mice exhibit a spontaneous tendency to explore novelty in free-exploratory paradigms, and we evaluated the ability of recognizing a previously presented environment versus a novel one, by measuring the difference in the exploration time of the familiar and novel environments. The boxes used were composed of two equal compartments (16 × 16 cm) connected by a central zone (16 × 5 cm). Boxes were made of grey Plexiglas and closed at the time of the experiments in order to be isolated from visual cues in the testing room. Light intensity in the boxes was approximately 15-20 lux. The two compartments showed two distinct spatial configurations (one rectangular or two triangular object(s) at one or two of the angles) and two distinct floor textures (grooves or peaks). The apparatus was equipped with infrared frame detectors to record the activity of the animals in each compartment. Mice were left 3 min in the central zone before being confined for 20 min in one of the two compartments ('familiar'). The mice were then placed back into their home cage for 15 min. During the following test phase, the animal was allowed to explore the whole box (central zone, familiar, and new compartment) for 15 min. The time spent in the familiar compartment and the time spent in the novel compartment were quantified. Before starting the experiments, we previously verified, in separate groups of animals, that i) C57B1/6J mice show a robust preference for the novel compartment when tested with no delay between the confinement and the test phases ii) that C57B16/J mice are able to discriminate both environments and iii) the absence of spontaneous preference for one of the compartments (data not shown). Mice were tested 7 months post injection (p.i). Data were analyzed with a paired two-tailed Student's t-test. Results are presented as mean± standard error of the mean.

*Novel object recognition (NOR).* Recognition memory was also evaluated with a novel object recognition task using a rectangular open field (50 × 25 cm) made of transparent Plexiglas. Before starting the experiment, we verified that mice did not display a preference among the different objects used. Mice were subjected to a 10 min training session in which they were allowed to explore two copies of the same object (A or B). Mice were placed back in their home cage for a retention interval of 15 min, then tested in a session of 10 min with two objects, one familiar and one novel (A or B). Light intensity in the room was approximately 50 lux. Each session was recorded and the time spent exploring the object was evaluated by an operator blinded to mouse group. The use of object A or B as familiar or novel object was randomized among mice to avoid any bias. Mice were tested 15 months p.i. Data were plotted as total exploration time and recognition percentage [TimeNovel / (TimeNovel + _{TimeFamiliar)} × 100] and analyzed with an unpaired (for recognition percentage) or paired (for total time) two-tailed Student's t-test. Results are presented as mean± standard error of the mean.

*Light-dark box (LDB)*: Anxiety was evaluated with the LDB paradigm using a rectangular cage (22 × 16 cm) made of Plexiglas, divided in two sections of equal size, the lit compartment with white walls and the dark with black walls. An illumination of 500 lux was applied to the lit side while the dark side was protected from light (about 2-4 lux in the dark compartment). Mice were placed in the dark compartment and allowed to freely explore the box for 5 min. The cage was place on an infrared board and movies were taken with an infrared camera. Transitions from one section to the other of the box and total time spent in each compartment were quantified with the software EthoVision XT (Noldus Information Technology, Wageningen, Netherlands). Mice were tested 12 months p.i. Data were analyzed with an unpaired two-tailed Student's t-test for both percentage spent in lit compartment [TimeLit / (TimeLit + _{TimeDark)} × 100] and number of transitions. Results are plotted as mean ± standard error of the mean.

For all behavioral tests, all apparatus were washed with water between individual sessions.

*Immunostaining.* Mice were deeply anesthetized with a lethal dose of Ketamine/Xylazine before intracardiac perfusion with ice cold PBS first, followed by PFA (Sigma-Aldrich, Saint Louis, MO, USA) 4% in PBS. The brains were collected and post-fixed overnight in PFA 4%. Coronal brain slices of 50 µm thickness were obtained using a vibratome (Leica Microsystems, Wetzlar, Germany). Slices were incubated in 10% NGS and 0.2% Triton X100 in PBS for one hour, then washed in PBS and incubated with primary antibodies: anti-GFP rabbit 1:2000 (Life Technologies, Invitrogen, Carlsbad, CA, USA) and VHH V31-1 kindly provided by Pierre Lafaye (Lafaye et al., 2009). We used an anti-His antibody (Sigma-Aldrich, Saint Louis, MO, USA) in order to amplify the VHH signal. The fluorophore-conjugated secondary antibodies used were Cy3-conjugated anti-mouse and Cy2-conjugated anti-rabbit, (Jackson Immunoresearch, West Grove, PA, USA) at a dilution of 1:500. After DAPI (Sigma-Aldrich, Saint Louis, MO, USA) incubation, the slices were mounted on slides and fixed with ProLong Gold Antifade Reagent (Life Technologies, Molecular Probes, Carlsbad, CA, USA). Quantification of fluorescence intensity was performed with Icy software (de Chaumont et al., 2012) (n=20, 10 GFP injected mice and 10 hAPP-SLA injected mice). Three slices from each mouse on the anterior posterior axis were analysed. Fluorescence intensity was normalized on ROI (region of interest) area. Fluorescent intensity/ROI area was used as index of number of cells transfected with the vector in the targeted area.

Primers. Table 6 lists the primers used in the present work. The mutations added are highlighted in bold, in primers used for directed PCR mutagenesis. Fw: forward primer; Rev: Reverse primer.

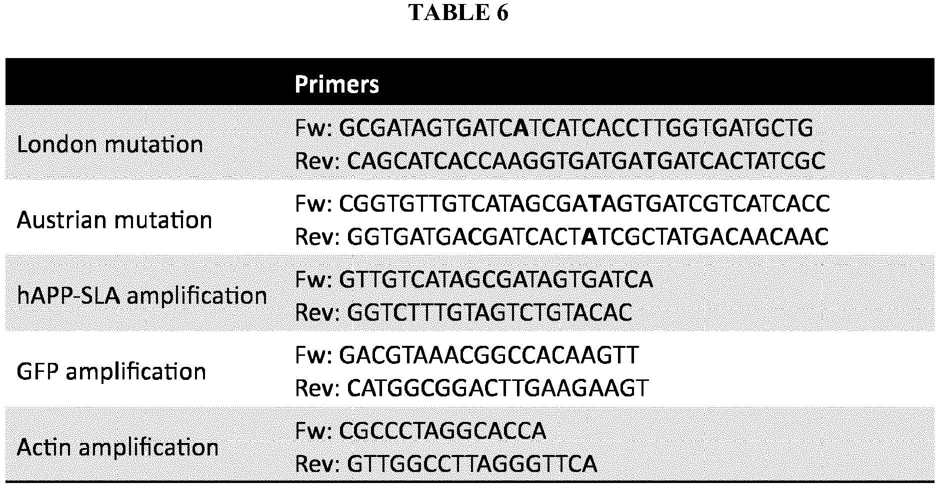

These primer sequences are also referred to in the listing sequence as SEQ ID NO :1 - 10.

### Example 2: The lentiviral vector stably expresses hAPP-SLA

We generated a lentiviral vector encoding the human sequence of the APP harbouring three pathogenic mutations, the Swedish double mutation (KM670/671NL) (Mullan et al., 1992), the London mutation (V717I) (Goate et al., 1991) and the Austrian mutation (T714I) (Kumar-Singh et al., 2000) (Fig. 1A). To study the effects of the expression of this protein in the brain, we stereotactically injected the viral particles into the dentate gyrus (DG) of 12 week-old WT mice. The correct expression of the transgene was verified one month post-injection (p.i.) by amplification of mRNA molecules encoding for hAPP-SLA and GFP. The APP sequence was amplified in the APP-WT group and not in GFP-WT group (Fig. 1B), while the GFP sequence was amplified in both groups (data not shown). We then evaluated the diffusion of the virus in the targeted area, using GFP protein staining as a marker. The GFP staining (green) showed a widespread diffusion in the DG (Fig. 1D), with little spreading of the staining in the surrounding regions of CA1 (Fig. 1D, arrow head) and CA3 (Fig. 1D, arrow). To further investigate the extension of the viral infection we quantified GFP fluorescence intensity/ROI area in DG granular cell layer, and CA1 and CA3 pyramidal cell layers. The quantification confirmed that the majority of the fluorescent cells were located in the DG (3-fold higher compared to CA1 and CA3) (Fig. 1C). The viral infection was therefore very much restricted to this area. In addition, a stable expression of hAPP-SLA and GFP was obtained, that lasted up to 15 months p.i.

### Example 3: In vivo hAPP-SLA expression induces a memory deficit

Behavioural tasks were carried out to assess recognition memory performance of mice expressing hAPP-SLA specifically in the DG. Mice (7 months p.i.) were tested in the NPR paradigm. The analysis of total time of exploration for the novel and familiar compartments during the test phase revealed a significant (Student's t-test) preference for the novel compartment in GFP-WT (p = 0.0003) (Fig. 2A), while APP-WT did not show any preference for novel or familiar compartment (p = 0.3) (Fig. 2B). We then performed the NOR task on mice 15 months p.i. The analysis of total time of exploration during the test session (Student's t-test) showed that both GFP-WT and APP-WT spent significantly more time exploring the novel object (p = 0.0002 and p = 0.047, respectively) (Fig. 2C and 2D, respectively). However, the analysis of the recognition percentage for the same set of data showed that GFP-WT spent significantly more time exploring the novel object compared to APP-WT (p = 0.0032) (Fig. 2E), revealing the presence of a memory deficit in the APP-WT group. In addition, we analyzed the locomotor behaviour during the NOR habituation phase: APP-WT showed higher locomotor activity compared to GFP-WT (p = 0.0027) (Fig. 2F). Then we measured anxiety levels using the LDB paradigm. No differences were observed between both groups for the index of time spent in the lit compartment (p = 0.7) as well as for the number of transitions (p = 0.2) (Figs. 2G-H).

The transduction of hAPP-SLA in DG induced recognition memory deficits as supported by NPR and NOR tasks. A GFAP and Iba1 staining was performed on WT-APP,WT-GFP, andWT noninjected mice to investigate whether or not the observed behavioral deficit could be driven by neuroinflammation.We did not detect any glia or microglia activation in WT-APP compared with WT-GFP, meaning that the neuroinflammation does not play a role in the observed memory deficit. In addition, viral transduction did not induce any neuroinflammation, as shown in WT non-injected mice (data not shown).

### Example 4: Beta-2 KO mice are protected from hAPP-SLA induced memory impairment

To address the potential role of nAChR subunits in the phenotype previously observed in APP-WT, we injected the vector into the dentate gyrus (DG) of mice KO for beta-2 or alpha-7 subunits. In the NPR task (7 months p.i.), the GFP-beta-2 spent significantly more time exploring the novel compartment (p = 0.003) (Fig. 3A), this was also the case for APP-beta-2 (p = 0.017) (Fig. 3B). Similarly, in the NOR task (15 months p.i.) both the GFP-beta-2 and the APP-beta-2 displayed higher exploration of the novel object (p < 0.0001 and p = 0.0001, respectively) (Fig. 3C and Fig. 3D), with no differences between the two groups in the recognition index (p = 0.7) (Fig. 3E), meaning that beta-2 mice injected with hAPP-SLA did not exhibit the recognition memory deficit observed in APP-WT. The analysis of the locomotion during the NOR habituation phase showed no differences between GFP-beta-2 and APP-beta-2 (p = 0.2) (Fig. 3F). The light-dark box test showed no differences in anxiety levels measured as total time spent in the lit compartment (p = 0.52) and number of transitions (p = 0.17) between the APP-beta-2 and the GFP-beta-2 (Fig. 3G-H). Thus, the hAPP-SLA transduction in DG did not induce a memory deficit in beta-2 KO, meaning that the Abeta/beta2-nAChR interaction is required to drive the memory deficit in this model.

### Example 5: nAChR alpha-7 KO mice display a memory impairment independently of hAPP expression

The involvement of the alpha-7 nAChR subunit in AD pathology had been investigated in previous studies that obtained conflicting results (Dziewczapolski et al., 2009; Hernandez et al., 2010), reviewed in Lombardo and Maskos (Lombardo and Maskos, 2015). In our experimental conditions we found that, in the NPR task (7 months p.i.), both GFP-alpha-7 (p = 0.9) (Fig. 4A) and APP-alpha-7 (p = 0.3) (Fig. 4B) showed no difference in the exploration of the novel or familiar compartment, meaning that both groups displayed a memory deficit in this test. We then tested age matched non-injected mice to determine whether the memory deficit was related to the viral injection in this strain. Non-injected mice spent the same amount of time exploring novel and familiar compartments during the test session (p = 0.6) (Fig. 4C), confirming the presence of a constitutive recognition memory deficit in aged alpha-7 KO mice. In the NOR task (15 months p.i.) as well, both GFP-alpha-7 and APP-alpha-7 displayed a cognitive impairment (Fig. 4D-E). The analysis of the total exploration time during the NOR task showed that GFP-alpha-7 spent similar time exploring the familiar and the novel objects (p = 0.76) (Fig. 4D), while APP-alpha-7 spent significantly more time exploring the novel object compared to the familiar one (p = 0.01) (Fig. 4E). Yet the analysis of the recognition index showed no difference between GFP-alpha-7 and APP-alpha-7 (Fig. 4F). We found no difference in locomotor activity between the two groups (p = 0.29) (Fig. 4G). The LDB test revealed no difference between GFP-alpha-7 and APP-alpha-7 in the percentage of time spent in the lit compartment (p = 0.6) (Fig. 4H), while the test showed higher number of transitions in APP-alpha-7 compared to GFP-alpha-7 (p = 0.01) (Fig. 41).

### Example 6: The expression of hAPP-SLA drives Abeta synthesis and intracellular accumulation

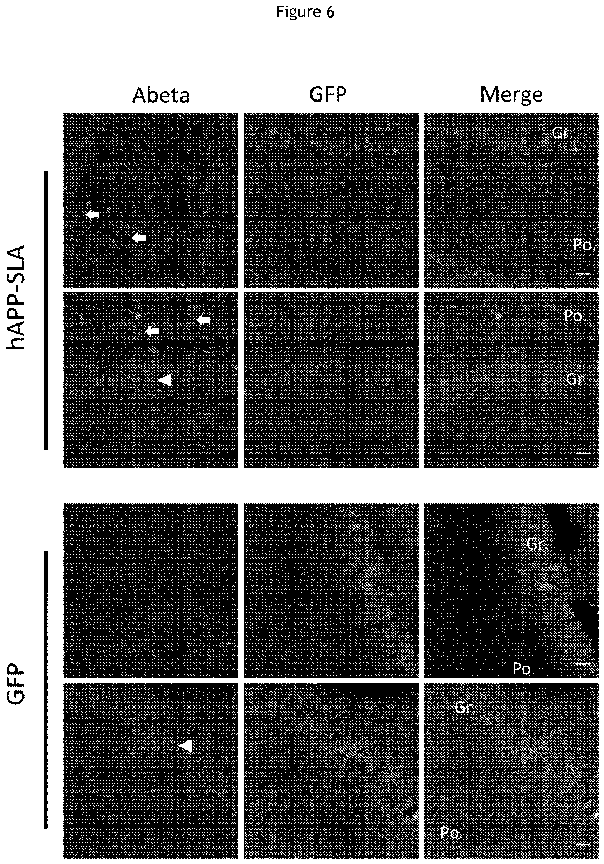

Several studies showed that in AD patients as well as in AD animal models, the appearance of the cognitive deficits precedes plaque deposition (Casas et al., 2004; Gouras et al., 2000; Kumar et al., 2013; Wirths et al., 2004). We investigated whether the expression of the hAPP-SLA protein was able to drive Abeta accumulation in our model. The presence of oligomeric Abeta in APP-WT was confirmed with the antibody VHH 31-1, specific for oligomeric forms of Abeta (Lafaye et al., 2009). Abeta staining was mainly found in the polymorphic layer of the DG (Fig. 5, arrows). In addition, Abeta synthesis was strictly related to hAPP-SLA expression, since Abeta oligomers were not observed in CA1, where there was no viral infection (Fig. 5). We then analyzed the presence of Abeta aggregates in the hippocampus of APP-beta-2 and APP-alpha-7. In APP-beta-2, a positive staining for Aβ oligomers using the VHH 31-1 antibody was found (Fig. 6, arrows). We observed intracellular Abeta staining in the polymorphic layer of the DG that was absent in GFP-beta-2 (Fig. 6). Immunofluorescence performed with VHH 31-1 on brain slices obtained from APP-alpha-7 mice also showed intracellular Abeta oligomers in the polymorphic layer (Fig. 6, arrows), while GFP-alpha-7 mice did not show Abeta staining.

Abeta intracellular accumulation in DG polymorphic layer was also confirmed with the rat monoclonal 7H3D6 antibody, also specific for oligomeric Abeta (Kumar et al., 2013). The antigen retrieval protocol required for this staining resulted in higher background noise and lower GFP signal. The signal in granular cell layer of WT-GFP and WT-APP was considered as background.

### Example 7: Analysis of APP-beta-2 and APP-alpha-7 Mice

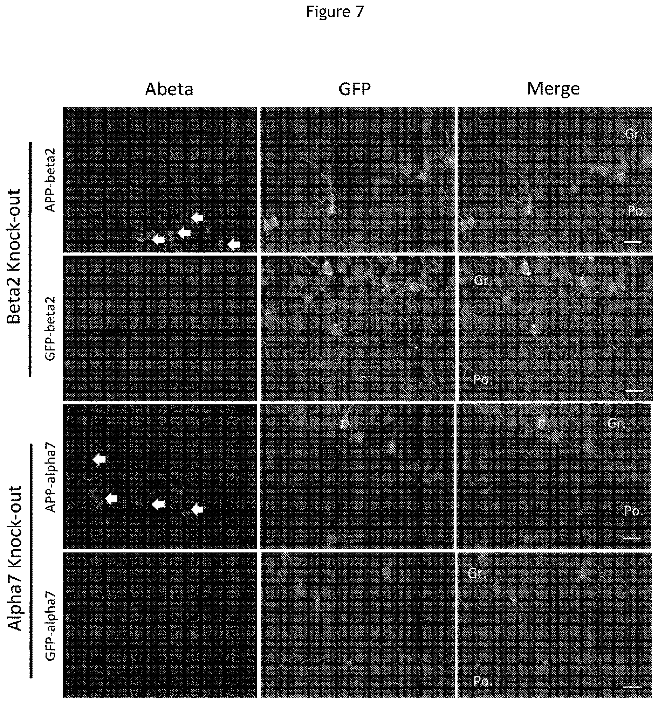

We analyzed the presence of Abeta aggregates in the hippocampus of APP-beta-2 and APP-alpha-7. In APP-beta-2, a positive staining for Abeta oligomers using the VHH 31-1 antibody was found (Fig. 7, arrows). We observed intracellular Abeta staining in the polymorphic layer of the DG that was absent in GFP-beta-2 (Fig. 7). Immunofluorescence performed with VHH 31-1 on brain slices obtained from APP-alpha-7 mice also showed intracellular Abeta oligomers in the polymorphic layer (Fig. 7, arrows), while GFP-alpha-7 mice did not show Abeta staining.

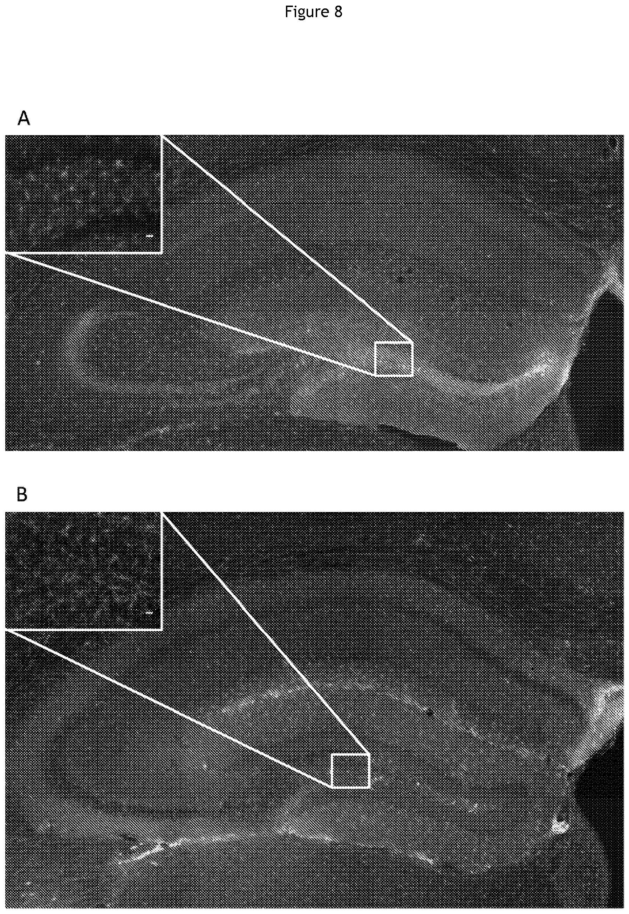

We performed an Iba1 staining on WT-APP and WT-GFP mice to investigate whether or not the behavioural deficit we observed could be driven by neuroinflammation. We did not detected any microglia activation in WT-APP compared to WT-GFP (Fig. 8).

### Example 8: Discussion on the results obtained with the lentivirus

In an attempt to investigate the implication of sub-structures of the hippocampus in amyloid pathology, we developed a novel mouse model based on the lentiviral delivery of a hAPP expressing vector in this area. The AD rodent models commonly used are transgenic organisms that constitutively express high levels of mutated hAPP in the whole brain, and throughout pre- and postnatal development, thereby not being appropriate to address this kind of questions. Current AD models rely on the expression of human proteins harbouring fAD associated mutations. This is to reproduce clinical and histopathological features of AD in murine models otherwise impossible to obtain.

However, none of these models allow the accurate expression of APP in a specific brain structure. Thus, the main advantage of the present model is to produce Abeta in the adult brain and in specific targeted area.

The vector we generated expresses the sequence of human APP harbouring three pathogenic mutations, the Swedish double mutation (KM670/671NL) (Mullan et al., 1992), the London mutation (V717I) (Goate et al., 1991) and the Austrian mutation (T714I) (Kumar-Singh et al., 2000). These three mutations were chosen for their close proximity to the secretase cleavage sites ((3-secretase for the Swedish and y-secretase for London and Austrian), and were reported to increase Abeta synthesis (Goate et al., 1991; Kumar-Singh et al., 2000; Mullan et al., 1992). The technology of lentiviral transduction was already used to generate models to study hAPP processing *in vitro* (Shaughnessy et al., 2005). The lentiviral vector allows the addition of the IRES-GFP sequence for an easy and rapid analysis of viral transfection efficiency, and diffusion in the targeted area. Lentiviral vectors remain precisely in the brain structure they target. We were thus able to infect specific sub-regions of the hippocampus, like the CA1 and CA3 pyramidal cell regions, and the dentate gyrus (DG).

Since memory is one of the first human cognitive functions affected in AD patients (Hildebrandt et al., 2013), we decided to target the hippocampus, and in particular the DG, which is a key region for memory formation (Morris, 2006; Squire et al., 2007). In addition, amyloid deposition in the hippocampus of AD patients was previously established (Braak and Braak, 1991). We quantified fluorescence intensity outside the targeted area and found 3-fold lower fluorescence intensity in CA1 and CA3 areas compared to DG, meaning that the majority of transfected cells are located in the selected area. There is potentially a diffusion of the vector solution from the needle to CA1 at the moment of the injection (Fig. 1D, arrow head), while the fluorescence in CA3 most probably originates from the projection of the DG through the mossy fiber pathway (Fig. 1D, arrow). We can conclude that the DG was efficiently targeted. Moreover, a single injection led to good vector diffusion within the hippocampus (Fig. 1D) and was able to induce a recognition memory deficit together with the accumulation of intracellular Abeta, no extracellular Abeta aggregates or plaques were found. These features are all associated with early-stage AD pathology (Kumar et al., 2013). For Abeta staining, we used a VHH derived from Alpaca antibodies (Hamers-Casterman et al., 1993) specific for oligomeric Abeta (Lafaye et al., 2009). The majority of cells positive for Abeta staining in DG did not express the lentivirus, with only a little portion of GFP positive cells exhibiting Abeta staining (Fig. 5, arrow head). However, Abeta synthesis was strictly confined to the lentivirus injection site, since in CA1, where there was no transgene expression, Abeta oligomers were absent (Fig. 5), and this was also the case in the whole hippocampus of GFP injected mice (Fig. 6). Thus, we have generated a novel model displaying some of the core features of AD in which it is possible to study changes induced by amyloid deposition in a restricted brain area.

The major advantage of the approach we used is that it is applicable to every animal model and strain or species. We decided to use it to study the implication of nAChRs in AD-like pathology. In this work we investigated the role of alpha-7 and beta-2 nAChR subunits. While alpha-7 had been shown to physically interact with Abeta (Wang et al., 2000a, 2000b), only a functional link *in vitro* between beta-2 and Abeta was reported, as Abeta was shown to inhibit beta2^{∗}-nAChR currents (Lamb et al., 2005; Pandya and Yakel, 2011). Our data strongly suggest that the lack of the beta-2 subunit prevents Abeta -induced memory loss, since beta-2 KO animals are protected from the recognition memory impairment induced by APP-SLA expression in control mice. To date, this is the first *in vivo* demonstration of a role for the beta-2 subunit in amyloid pathology. Previous studies extensively reported degeneration of the cholinergic system in AD, particularly the basal forebrain cholinergic cells that innervate the neocortex and hippocampus. Their degeneration in AD is associated with reduced levels of pre- and postsynaptic cholinergic markers in the cortex, hippocampus, and amygdala.

Electrophysiological and binding studies demonstrated that nAChRs are expressed in discrete parts of the hippocampus. Notably, in the DG both alpha-7 and beta-2 subunits are expressed in the molecular layer, in particular, in GABAergic interneurons (Frazier et al., 2003; Gahring and Rogers, 2008; Son and Winzer-Serhan, 2008), and alpha-7 nAChRs were reported to induce LTP in DG (Matsuyama et al., 2000). No nicotinic responses have been identified in dentate granule cells (Jones and Yakel, 1997), but hippocampal neurogenesis is altered in beta-2 KO mice (Harrist et al., 2004). Liu et al. (Liu et al., 2009) recently postulated the existence of an alpha-7 beta-2 nAChR heteromer in basal forebrain cholinergic neurons, that is also expressed in CA1 interneurons. This novel nAChR subtype is characterized by an high sensitivity to Abeta inhibition (Liu et al., 2012) and this interaction could be responsible for the memory deficit observed in AD. It has been reported that both beta-2 and alpha-7 subunits are important for working memory. Local infusion of alpha-7 nAChR and alpha-4 beta-2 nAChR antagonist (MLA and DHβE, respectively) in ventral hippocampus induces working memory impairments (Felix and Levin, 1997; Levin et al., 2002). Thus, nAChRs are key regulatory elements of hippocampal circuit activity (Frazier et al., 2003; Orr-Urtreger et al., 1997). As most nAChRs in the hippocampus and upper layers of cortex are expressed only in interneurons, nAChR- Abeta interaction could alter the electrophysiological activity of interneurons in the dentate gyrus. Abeta inhibition of nAChRs could interfere with their regulatory function, leading to neuronal hyperactivity in the hippocampus and subsequent memory impairment. In beta-2 KO, the absence of the subunit could be protective for the neurons by preventing nAChR- Abeta toxic interaction.

Previous *in vivo* work attempted to dissect the role of alpha-7 nAChRs in AD (Dziewczapolski et al., 2009; Hernandez et al., 2010). In both studies AD mouse models were crossed with alpha-7 nAChR null-mutants. However, these studies came to opposite conclusions, improvement and worsening, respectively, of AD-associated phenotypes, such as memory and synaptic deficits. In our experimental conditions, APP-alpha-7 displayed an inherent memory deficit that was independent of injection, making it impossible to conclude concerning the effect of Abeta accumulation in this line. The alpha-7 KO line had previously been characterized by Paylor et al. (Paylor et al., 1998). A large panel of behaviours like motor and sensory responses, locomotor activity in the open field, motor coordination, and spatial memory were investigated. These authors found no difference between alpha-7 KO and WT mice. More recent studies showed an attention deficit in the five choice serial reaction time task, but no memory impairment, in alpha-7 KO mice (Hoyle et al., 2006; Young et al., 2011, 2007, 2004). However, young mice were used, while, in our case, mice were 18 months old at the end of the experiments. To conclude, these studies have not been able to conclude on the precise role of the alpha-7 subunit.

### Example 9: Further analysis of hippocampal electrophysiology

Intracranial injections of these lentiviruses have been carried out in WT and genetically engineered mice as described in Lombardo et al (2016). These mice are KO for the alpha-7 subunit, the beta-2 subunuit or both the alpha-7 and beta-2 subunits (α7/β2 double KO).

The experimental procedures for generating these mice has been published in Koukouli et al, Nature Medicine, 2017, which is herein incorporated by reference.

Animals were aged 18 months when electrophysiology was carried out.

Hippocampal coronal slices (400 µm thickness) were prepared from +/- 2 year old mice. (wt-gfp: n=11(4 mice), wt-app: n=17 (7 mice), a7-gfp: n=14 (4 mice), a7-app: n=12 (5 mice), b2-gfp: n=15 (5 mice), b2-app: n= 17 (7 mice), b2a7-gfp n=13 (5 mice), b2a7-app n=16 (7 mice). Slices were cut in ice-cold slicing artificial cerebrospinal fluid (ACSF) containing the following (in mM): 93 NMDG, 93 HCl(adjust to pH= 7.2), 2.5 KCl, 1.2 NaH₂PO₄, 30 NaHCO₃, 20 Hepes, 25 Glucose, 12 NAC, 5 Sodium ascorbate, 2 Thiourea, 3 Sodium pyruvate, 10 MgSO₄, and 0.5 CaCl₂ (300 mOsm), and carboxygenated with 95%O₂/5%CO₂. Slices were allowed to recover for 1 h at RT in modified carboxygenated ACSF containing the following (in mM): 125 NaCl, 3 KCl, 1.25 NaH₂PO₄, 26 NaHCO₃, 10 glucose, 1.3 MgSO₄, 2.4 CaCl₂, and 0.01 glycine (300 mOsm). Slices were superfused with 1-2 ml/min carboxygenated ACSF at RT and additional carbogen was blown over the slice. Field excitatory post-synaptic potentials (fEPSPs) were recorded from multiple electrodes, using a planar 64-channel multi-electrode recording setup (MED64; Alpha MED Sciences), in the dendritic layer of the CA1 neurons after stimulation with an external stimulation electrode (440b isolator bipolar current stimulator) in the Schaffer collateral pathway. Based on the stimulus-response curve, a stimulation intensity that evoked the fEPSP with a magnitude of 50% of the maximum response was chosen. After at least 10 min of stable baseline recording at 0.05 Hz, long-term potentiation (LTP) was induced by 3 trains of 100 Hz stimulus (tetanus), separated by 20 s. The fEPSPs were recorded for 1 h after the tetanus. LTP was expressed as the change in average amplitude of the fEPSP taken from 40 to 50 min interval after LTP induction.

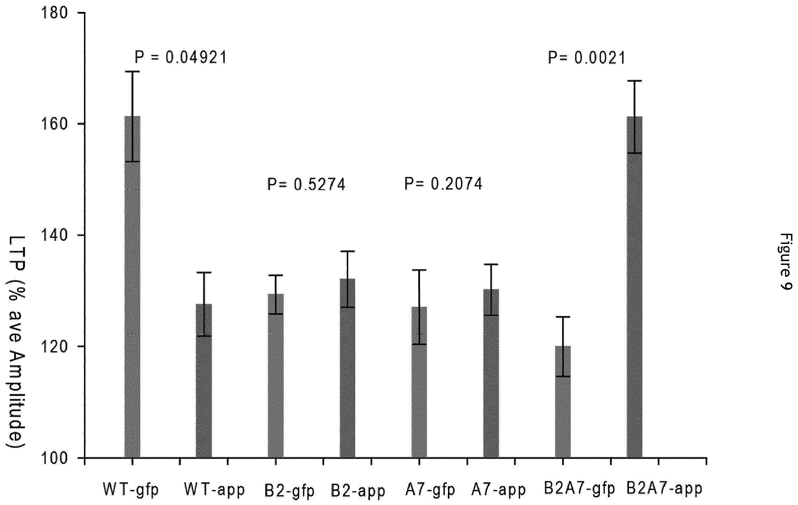

As shown on Figure 9, there is a baseline deficit in hippocampal LTP (long term potentiation, an electrophysiological correlate of memory formation) in single KO alpha-7 and beta-2 animals, and double KO alpha-7 beta-2 animals, as well as in WT mice expressing A beta.

However, double KO alpha7xbeta2 animals expressing A beta are completely normal. This indicates that blocking at the same time beta-2 and alpha-7 containing receptors is a promising means for impairing AD onset in the wild-type mouse.

### Example 10 : Construction of an AAV for replicating AD-like pathology

An adeno-associated virus (AAV) based model for replicating AD-like pathology was developed by the present inventors. It has been described in Koukouli et al, Aging 2016 which is herein incorporated by reference.

This AAV expresses large amounts of A beta. Cortical activity deficits were observed in injected mice after four weeks.

Unlike the lentiviruses used previously which target the hippocampal region of the brain, these Adeno-associated viral vectors-express the mutated Amyloid Precursor Protein (APP) in the mouse cortex. These mutant AAV-APP viruses cause neuronal accumulation of amyloid peptides and eventually amyloid plaques at 6 months post-injection (Koukouli et al, Aging 2016).

Intracranial injections of these AAV viruses have been carried out to the Prelimbic Cortex of WT and genetically engineered mice. These mice are KO for the alpha-7 subunit, the beta-2 subunit or both the alpha-7 and beta-2 subunits (α7/β2 double KO).

The experimental procedures for generating these mice has been published in Koukouli et al, Nature Medicine, 2017, which is herein incorporated by reference.

The AAV virus was injected into these KO mice. This virus targets particular brain areas, although the transgenic mouse models express plaques everywhere.

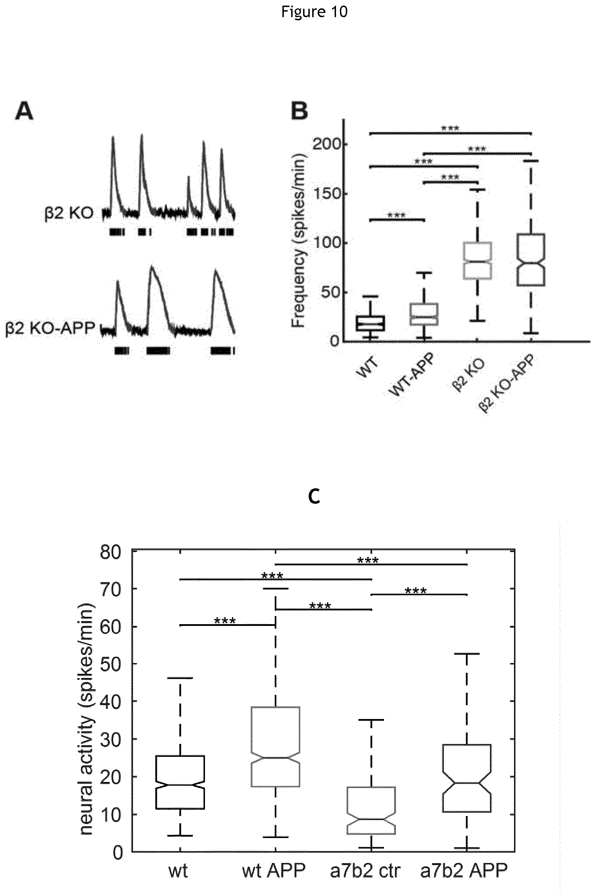

The results are disclosed on figure 10. Cortical activity was measured as described in Koukouli et al, Aging 2016. Figure 10 A and B shows the results obtained in the cortex of beta-2 KO mice.

Abeta expression does not alter the level of cortical activity exhibited by the beta-2 KO mouse under saline treatment, i.e. the Abeta does not have an effect on mice lacking the beta-2 subunit, as found in the hippocampal experiments.

Figure 10 C shows the results obtained in alpha-7 / beta-2 double KO mice. Similar to the analysis in hippocampus performed earlier, it has been observed that the cortical activity in the double KO alpha-7 beta-2 mice is completely normal in the presence of Abeta (see right column in figure 10C).

A further experiment has been carried out: Abeta was expressed by administering the AAV-hAPP virus in wild-type mice, and two antagonists of alpha-7 and beta-2 nAChRs were administered by mini-pumps, as described in Besson et al, PNAS 2007.

The alpha-7 antagonist was MLA and the beta-2 antagonist was Dihydro-β-erythroidine (DHβE).

*Surgical Implantation of Minipumps.* Mice were slightly anesthetized with a xylazine and ketamine combination (15% xylazine/ 2.5% ketamine/82.5% PBS). Osmotic minipumps (model 2004, ALZET; DURECT) were implanted s.c. at the nape of the neck. They delivered either a cocktail of the two antagonists in a saline solution (pH=7) at a constant rate (1 mg/kg per day for DHβE and 1 mg/kg per day for MLA) or a controle saline solution (pH=7) and were left in place for up to four weeks.

The cortical activity was measured once a week, for four weeks.

The results are displayed on Figure 12. WT mice, controls, and WT mice injected with the AAV-hAPP vector were compared. Groups were treated with saline, control, or the two antagonists (nic). Treatment with the two antagonists lowered cortical activity in AAV-hAAP mice compared to saline control.

### REFERENCES CITED

Abdrakhmanova et al., 2-Fluoro-3-(4-nitro-phenyl)deschloroepibatidine Is a Novel Potent Competitive Antagonist of Human Neuronal alpha 4 beta 2 nAChRs Mol Pharmacol 69:1945-1952,2006
Aracava Y, Pereira EF, Maelicke A, Albuquerque EX (March 2005). "Memantine blocks alpha7∗ nicotinic acetylcholine receptors more potently than n-methyl-D-aspartate receptors in rat hippocampal neurons". J Pharmacol Exp Ther. 312 (3): 1195-205.
Hugo R. Arias, et al., "Positive and Negative Regulation of Nicotinic Receptors," Adv. Protein Chem. Struct. Biol., 80 (2010) 153-203.
Auld, D.S., Kornecook, T.J., Bastianetto, S., Quirion, R., 2002. Alzheimer's disease and the basal forebrain cholinergic system: relations to beta-amyloid peptides, cognition, and treatment strategies. Prog. Neurobiol. 68, 209-245. doi:10.1016/S0301-0082(02)00079-5
Baranowska U, Göthert M, Rudz R, Malinowska B. Methanandamide allosterically inhibits in vivo the function of peripheral nicotinic acetylcholine receptors containing the alpha 7-subunit. J Pharmacol Exp Ther. 2008 Sep;326(3):912-9. doi: 10.1124/jpet.108.140863. Epub 2008 Jun 20.
Besson et al, Long-term effects of chronic nicotine exposure on brain nicotinic receptors. PNAS 2007 May 8;104(19)
Braak, H., Braak, E., 1991. Neuropathological stageing of Alzheimer-related changes. Acta Neuropathol. (Berl.) 82, 239-259.
Brandon et al. Negative allosteric modulators that target human α4β2 neuronal nicotinic receptors Journal of Pharmacology and Experimental Therapeutics; vol. 334; nb. 3; (2010); p.761 - 774
Bridges et al. UCI-30002 - a novel nAChR negative allosteric modulator Current topics in medicinal chemistry; vol. 8; nb. 3; (2008); p. 268 - 268
Briggs CA, McKenna DG (April 1996). "Effect of MK-801 at the human alpha 7 nicotinic acetylcholine receptor". Neuropharmacology. 35 (4): 407-14.
Buckingham S.D., Jones A.K., Brown L.A., Sattelle D.B., 2009 Nicotinic acetylcholine receptor signalling: roles in Alzheimer's disease and amyloid neuroprotection. Pharmacol Rev. 61(1):39-61
Cartier et al (1996) A new α-conotoxin which targets α3β2 nicotinic acetylcholine receptors. J.Biol.Chem. 271 7522. PMID: 8631783
Casas, C., Sergeant, N., Itier, J.-M., Blanchard, V., Wirths, O., van der Kolk, N., Vingtdeux, V., van de Steeg, E., Ret, G., Canton, T., Drobecq, H., Clark, A., Bonici, B., Delacourte, A., Benavides, J., Schmitz, C., Tremp, G., Bayer, T.A., Benoit, P., Pradier, L., 2004. Massive CA1/2 Neuronal Loss with Intraneuronal and N-Terminal Truncated Abeta42 Accumulation in a Novel Alzheimer Transgenic Model. Am. J. Pathol. 165, 1289-1300. doi:10.1016/S0002-9440(10)63388-3
Choo YM, Lee BH, Lee KS, Kim BY, Li J, Kim JG, Lee JH, Sohn HD, Nah SY, Jin BR. Pr-lynx1, a modulator of nicotinic acetylcholine receptors in the insect. Mol Cell Neurosci. 2008 Jun; 38(2):224-35.
Covernton PJO, Connolly JG (1997) Differential modulation of rat neuronal nicotinic receptor subtypes by acute application of ethanol. Br J Pharmacol 122:1661-1668.
John W. Daly, "Nicotinic Agonists, Antagonists, and Modulators From Natural Sources," Cellular and Molecular Neurobiology, Vol. 25, Nos. 3/4, June 2005, 513-552.
Damaj, M. I.; Patrick, G. S.; Creasy, K. R.; Martin, B. R. (1997). "Pharmacology of lobeline, a nicotinic receptor ligand". The Journal of Pharmacology and Experimental Therapeutics. 282 (1): 410-9.
Damaj et al (1995) In vivo pharmacological effects of dihydro-β-erythroidine, a nicotinic antagonist, in mice. Psychopharmacology 117 67. PMID: 7724704.
Danober L et al, Pharmacological profile of S76892, a new ligand at nicotinic α7-subtype receptors. Biochemical Pharmacology, abstract of 2015
Darvas et al. Modulation of the Ca2+ conductance of nicotinic acetylcholine receptors by Lypd6 European Neuropsychopharmacology (2009) 19, 670-681
De Chaumont, F., Dallongeville, S., Chenouard, N., Hervé, N., Pop, S., Provoost, T., Meas-Yedid, V., Pankajakshan, P., Lecomte, T., Le Montagner, Y., Lagache, T., Dufour, A., Olivo-Marin, J.-C., 2012. Icy: an open bioimage informatics platform for extended reproducible research. Nat. Methods 9, 690-696. doi:10.1038/nmeth.2075
Decker et al., Erysodine, a competitive antagonist at neuronal nicotinic acetylcholine Receptors, European Journal of Pharmacology 280 (1995) 79-89
Dubois B, Feldman HH, Jacova C, Cummings JL, Dekosky ST, Barberger-Gateau P, Delacourte A, Frisoni G, Fox NC, Galasko D, Gauthier S, Hampel H, Jicha GA, Meguro K, O'Brien J, Pasquier F, Robert P, Rossor M, Salloway S, Sarazin M, de Souza LC, Stern Y, Visser PJ, Scheltens P. 2010 Revising the definition of Alzheimer's disease: a new lexicon. Lancet Neurol. 9(11), 1118-27. doi: 10.1016/S1474-4422(10)70223-4
Dull, T., Zufferey, R., Kelly, M., Mandel, R.J., Nguyen, M., Trono, D., Naldini, L., 1998. A Third-Generation Lentivirus Vector with a Conditional Packaging System. J. Virol. 72, 8463-8471.
Dziewczapolski, G., Glogowski, C.M., Masliah, E., Heinemann, S.F., 2009. Deletion of the alpha-7 Nicotinic Acetylcholine Receptor Gene Improves Cognitive Deficits and Synaptic Pathology in a Mouse Model of Alzheimer's Disease. J. Neurosci. 29, 8805-8815. doi:10.1523/JNEUROSCI.6159-08.2009
Felix, R., Levin, E.D., 1997. Nicotinic antagonist administration into the ventral hippocampus and spatial working memory in rats. Neuroscience 81, 1009-1017. doi:10.1016/S0306-4522(97)00224-8
Frazier, C.J., Strowbridge, B.W., Papke, R.L., 2003. Nicotinic Receptors on Local Circuit Neurons in Dentate Gyrus: A Potential Role in Regulation of Granule Cell Excitability. J. Neurophysiol. 89, 3018-3028. doi:10.1152/jn.01036.2002
Frost, B., Diamond, M.I., 2010. Prion-like mechanisms in neurodegenerative diseases. Nat. Rev. Neurosci. 11, 155-159. doi:10.1038/nrn2786
Fry et al. Isolation of a Neurotoxin (α-colubritoxin) from a Nonvenomous Colubrid: Evidence for Early Origin of Venom in Snakes Journal of Molecular Evolution, 2003, vol.57, #4 p.446-452
Fujii et al. SLURP-1, an endogenous α7 nicotinic acetylcholine receptor allosteric ligand, is expressed in CD205+ dendritic cells in human tonsils and potentiates lymphocytic cholinergic activity Journal ofNeuroimmunology 267 (2014) 43-49
Gahring, L.C., Rogers, S.W., 2008. Nicotinic acetylcholine receptor expression in the hippocampus of 27 mouse strains reveals novel inhibitory circuitry. Hippocampus 18, 737-749. doi:10.1002/hipo.20430
Galya R. Abdrakhmanova, Bruce E. Blough, Carey Nesloney, Hernán A. Navarro, M. Imad Damaj, and F. Ivy Carroll In vitro and in vivo characterization of a novel negative allosteric modulator of neuronal nAChRs Neuropharmacology. 2010 November ; 59(6): 511-517
Goate, A., Chartier-Harlin, M.-C., Mullan, M., Brown, J., Crawford, F., Fidani, L., Giuffra, L., Haynes, A., Irving, N., James, L., Mant, R., Newton, P., Rooke, K., Roques, P., Talbot, C., Pericak-Vance, M., Roses, A., Williamson, R., Rossor, M., Owen, M., Hardy, J., 1991. Segregation of a missense mutation in the amyloid precursor protein gene with familial Alzheimer's disease. Nature 349, 704-706. doi:10.1038/349704a0
Gonzalez-Cestari et al. Effect of Novel Negative Allosteric Modulators of Neuronal Nicotinic Receptors on Cells Expressing Native and Recombinant Nicotinic Receptors: Implications for Drug Discovery, The journal of pharmacology and experimental therapeutics, 2009 Vol. 328, No.2
Gotti, C., Moretti, M., Bohr, I., Ziabreva, I., Vailati, S., Longhi, R., Riganti, L., Gaimarri, A. McKeith, I.G., Perry, R.H., Aarsland, D., Larsen, J.P., Sher, E., Beattie, R., Clementi, F., Court, J.A., 2006. Selective nicotinic acetylcholine receptor subunit deficits identified in Alzheimer's disease, Parkinson's disease and dementia with Lewy bodies by immunoprecipitation. Neurobiol. Dis. 23, 481-489. doi:10.1016/j.nbd.2006.04.005.
Gotti et al, Heterogeneity and complexity of native brain nicotinic receptors. Biochem Pharmacol. 2007 Oct 15;74(8):1102-11.
Gottschalk A , Schafer WR. Visualization of integral and peripheral cell surface proteins in live Caenorhabditis elegans. J Neurosci Methods. 2006 Jun 30; 154(1-2):68-79. Epub 2006 Feb 8.
Gouras, G.K., Tsai, J., Naslund, J., Vincent, B., Edgar, M., Checler, F., Greenfield, J.P., Haroutunian, V., Buxbaum, J.D., Xu, H., Greengard, P., Relkin, N.R., 2000. Intraneuronal Abeta42 Accumulation in Human Brain. Am. J. Pathol. 156, 15-20.
Hamers-Casterman, C., Atarhouch, T., Muyldermans, S., Robinson, G., Hamers, C., Songa, E.B., Bendahman, N., Hamers, R., 1993. Naturally occurring antibodies devoid of light chains. Nature 363, 446-448. doi:10.1038/363446a0
Harrist, A., Beech, R.D., King, S.L., Zanardi, A., Cleary, M.A., Caldarone, B.J., Eisch, A., Zoli, M., Picciotto, M.R., 2004. Alteration of hippocampal cell proliferation in mice lacking the beta-2 subunit of the neuronal nicotinic acetylcholine receptor. Synap. N. Y. N 54, 200-206. doi:10.1002/syn.20081
Henderson et al., Negative allosteric modulators that target human alpha-4 beta-2 neuronal nicotinic receptors, J Pharmacol Exp Ther. 2010 334(3):761-74
Henderson BJ, Orac CM, Maciagiewicz I, Bergmeier SC, McKay DB. 3D-QSAR and 3D-QSSR models of negative allosteric modulators facilitate the design of a novel selective antagonist of human α4β2 neuronal nicotinic acetylcholine receptors. Bioorg Med Chem Lett. 2012 Feb 15;22(4):1797-813.
Hernandez, C.M., Kayed, R., Zheng, H., Sweatt, J.D., Dineley, K.T., 2010. Loss of α7 Nicotinic Receptors Enhances (3-Amyloid Oligomer Accumulation, Exacerbating Early-Stage Cognitive Decline and Septohippocampal Pathology in a Mouse Model of Alzheimer's Disease. J. Neurosci. 30, 2442-2453. doi:10.1523/JNEUROSCI.5038-09.2010
Hijazi et al. Boudin is required for septate junction organisation in Drosophila and codes for a diffusible protein of the Ly6 superfamily Development 136, 2199-2209 (2009)
Hildebrandt, H., Fink, F., Kastrup, A., Haupts, M., Eling, P., 2013. Cognitive Profiles of Patients with Mild Cognitive Impairment or Dementia in Alzheimer's or Parkinson's Disease. Dement. Geriatr. Cogn. Disord. EXTRA 3, 102-112. doi:10.1159/000348350
Hilmas, C.; Pereira, EFR; Alkondon, M.; Rassoulpour, A.; Schwarcz, R.; Albuquerque, E.X. (2001). "The Brain Metabolite Kynurenic Acid Inhibits α7 Nicotinic Receptor Activity and Increases Non-α7 Nicotinic Receptor Expression: Physiopathological Implications". J. Neurosci. 21 (19): 7463-7473.
Hoyle, E., Genn, R.F., Fernandes, C., Stolerman, I.P., 2006. Impaired performance of alpha-7 nicotinic receptor knockout mice in the five-choice serial reaction time task. Psychopharmacology (Berl.) 189, 211-223. doi:10.1007/s00213-006-0549-2
Janes, α-Conotoxins as selective probes for nicotinic acetylcholine receptor subclasses Current Opinion in Pharmacology 2005, 5:280-292
JasKiran K. Gill-Thind‡1, Persis Dhankher§2, Jarryl M. D'Oyley§2, Tom D. Sheppard§3, and Neil S. Millar Structurally Similar Allosteric Modulators of 7 Nicotinic Acetylcholine Receptors Exhibit Five Distinct Pharmacological Effects THE JOURNAL OF BIOLOGICAL CHEMISTRY VOL. 290, NO.6, pp. 3552-3562, February 6, 2015
Jensen MM, Arvaniti M, Mikkelsen JD, Michalski D, Pinborg LH3, Härtig W,Thomsen MS.Prostate stem cell antigen interacts with nicotinic acetylcholine receptors and is affected in Alzheimer's disease. Neurobiol Aging. 2015 Apr;36(4):1629-38.
Jones, S., Yakel, J.L., 1997. Functional nicotinic ACh receptors on interneurones in the rat hippocampus. J. Physiol. 504 ( Pt 3), 603-610.
Koukouli et Maskos, The multiple roles of the α7 nicotinic acetylcholine receptor in modulating glutamatergic systems in the normal and diseased nervous system. Biochemical Pharmacology 2015 Oct 15;97(4):378-87
Koukouli et al, Nicotine reverses hypofrontality in animal models of addiction and schizophrenia. Nature Medicine, 2017 Mar;23(3):347-354.
Koukouli et al, Early and progressive deficit of neuronal activity patterns in a model of local amyloid pathology in mouse prefrontal cortex. Aging 2016 Dec 20;8(12):3430-3449
Kumar-Singh, S., De Jonghe, C., Cruts, M., Kleinert, R., Wang, R., Mercken, M., Strooper, B.D., Vanderstichele, H., Löfgren, A., Vanderhoeven, I., Backhovens, H., Vanmechelen, E., Kroisel, P.M., Broeckhoven, C.V., 2000. Nonfibrillar diffuse amyloid deposition due to a gamma 42-secretase site mutation points to an essential role for N-truncated Abeta42 in Alzheimer's disease. Hum. Mol. Genet. 9, 2589-2598. doi:10.1093/hmg/9.18.2589
Kumar, S., Wirths, O., Theil, S., Gerth, J., Bayer, T.A., Walter, J., 2013. Early intraneuronal accumulation and increased aggregation of phosphorylated Abeta in a mouse model of Alzheimer's disease. Acta Neuropathol. (Berl.) 125, 699-709. doi:10.1007/s00401-013-1107-8
Kuwabara H, Spivak CE, Xiao Y, Kellar K, Ravert HT, Kumar A, Alexander M, Hilton J, Wong DF, Dannals RF, Horti AG. Discovery of (-)-7-methyl-2-exo-[3'-(6-[18F]fluoropyridin-2-yl)-5'-pyridinyl]-7-azabicyclo[2.2.1]heptane, a radiolabeled antagonist for cerebral nicotinic acetylcholine receptor (alpha4beta2-nAChR) with optimal positron emission tomography imaging properties. J Med Chem. 2008 Aug 14;51(15):4751-64.
Lafaye, P., Achour, I., England, P., Duyckaerts, C., Rougeon, F., 2009. Single-domain antibodies recognize selectively small oligomeric forms of amyloid beta, prevent Abeta-induced neurotoxicity and inhibit fibril formation. Mol. Immunol. 46, 695-704. doi:10.1016/j.molimm.2008.09.008
Lamb, P.W., Melton, M.A., Yakel, J.L., 2005. Inhibition of neuronal nicotinic acetylcholine receptor channels expressed in Xenopus oocytes by beta-amyloid1-42 peptide. J. Mol. Neurosci. 27, 13-21. doi:10.1385/JMN:27:1:013.
Lee RH et al. Memantine inhibits α3β2-nAChRs-mediated nitrergic neurogenic vasodilation in porcine basilar arteries. PLoS One. 2012;7(7):e40326.
Levin, E.D., Bradley, A., Addy, N., Sigurani, N., 2002. Hippocampal alpha-7 and alpha-4 beta-2 nicotinic receptors and working memory. Neuroscience 109, 757-765. doi:10.1016/S0306-4522(01)00538-3
Levitin et al. PATE Gene Clusters Code for Multiple, Secreted TFP/Ly-6/uPAR Proteins That Are Expressed in Reproductive and Neuron-rich Tissues and Possess Neuromodulatory Activity The Journal Of Biological Chemistry Vol. 283, NO. 24, pp. 16928-16939, June 13, 2008
Liu, Q., Huang, Y., Shen, J., Steffensen, S., Wu, J., 2012. Functional alpha-7 beta-2 nicotinic acetylcholine receptors expressed in hippocampal interneurons exhibit high sensitivity to pathological level of amyloid betapeptides. BMC Neurosci. 13, 155-166. doi:10.1186/1471-2202-13-155
Liu, Q., Huang, Y., Xue, F., Simard, A., DeChon, J., Li, G., Zhang, J., Lucero, L., Wang, M., Sierks, M., Hu, G., Chang, Y., Lukas, R.J., Wu, J., 2009. A Novel Nicotinic Acetylcholine Receptor Subtype in Basal Forebrain Cholinergic Neurons with High Sensitivity to Amyloid Peptides. J. Neurosci. 29, 918-929. doi:10.1523/JNEUROSCI.3952-08.2009
Liu Z, Cao G, Li J, Bao H, Zhang Y. Identification of two Lynx proteins in Nilaparvata lugens and the modulation on insect nicotinic acetylcholine receptors. J Neurochem. 2009 Sep;110(5):1707-14.
Lombardo, S., Maskos, U., 2015. Role of the nicotinic acetylcholine receptor in Alzheimer's disease pathology and treatment. Neuropharmacology 96, 255-262. doi:10.1016/j.neuropharm.2014.11.018
Lombardo S, Catteau J, Besson M, Maskos U. A role for β2∗ nicotinic receptors in a model of local amyloid pathology induced in dentate gyrus. Neurobiol Aging. 2016 Oct;46:221-34 C. LOUIS, N. ROGE, J.-Y. THOMAS, G. DAS DORES, A. HUGOT, M.-H. GANDON, V. BERTAINA-ANGLADE5, A. KRAZEM, D.BÉRACOCHÉ A, D. BERTRAND, D. RIMET, T. PILLOT, M. BERTRAND, I. BOTEZ, J.-M. FOURQUEZ, L. DANOBER, P. LESTAGE Pharmacological profile of S 76892, a new ligand at nicotinic α7-subtype Receptors Program#/Poster#: 54.02/H25 Presenter at Poster: Sat, Oct. 17, 2015 Neurosciences 2015
Low et al., Three dimensional structure of erabutoxin b neurotoxic protein: Inhibitor of acetylcholine receptor PNAS Vol. 73, No. 9, pp. 2991-2994, September 1976
Luetje, C.W., 2004. Getting past the asterisk: the subunit composition of presynaptic nicotinic receptors that modulate striatal dopamine release. Mol. Pharmacol. 65, 1333-1335.
Lukas, R.J., Changeux, J.-P., Le Nove' re, N., Albuquerque, E.X., Balfour, D.J.K., Berg, D.K., Bertrand, D., Chiappinelli, A.A., Clarke, P.B.S., Collins, A.C., Dani, J.A., Grady, S.R.,
Kellar, K.J., Lindstrom, J.M., Marks, M.J., Quik, M., Taylor, P.W., Wonnacott, S., 1999. International union of pharmacology. XX. Current status of the nomenclature for nicotinic acetylcholine receptors and their subunits. Pharmacol. Rev. 51, 397-401.
Lyukmanova EN, Shulepko MA, Kudryavtsev D, Bychkov ML, Kulbatskii DS, Kasheverov IE, Astapova MV , Feofanov AV, Thomsen MS, Mikkelsen JD, Shenkarev ZO, Tsetlin VI, Dolgikh DA, Kirpichnikov MP. Human Secreted Ly-6/uPAR Related Protein-1 (SLURP-1) Is a Selective Allosteric Antagonist of α7 Nicotinic Acetylcholine Receptor. PLoS One. 2016 Feb 23; 11(2):e0149733.
Matsuyama, S., Matsumoto, A., Enomoto, T., Nishizaki, T., 2000. Activation of nicotinic acetylcholine receptors induces long-term potentiation in vivo in the intact mouse dentate gyrus.
Eur. J. Neurosci. 12, 3741-3747. doi:10.1046/j.1460-9568.2000.00259.x Neil S. Millar, et al., "Diversity of vertebrate nicotinic acetylcholine receptors," Neuropharmacology 56 (2009) 237-246.
Miwa et al. Lynx1, an Endogenous Toxin-like Modulator of Nicotinic Acetylcholine Receptors in the Mammalian CNS, Neuron, 1999 Vol. 23, 105-114
Miwa et al., Optimizing cholinergic tone through lynx modulators of nicotinic receptors: implications for plasticity and nicotine addiction. Physiology (Bethesda), 2012 27(4):187-99
Moaddel, Ruin; Abdrakhmanova, Galia; Kozak, Joanna; Jozwiak, Krzysztof; Toll, Lawrence; Jimenez, Lucita; Rosenberg, Avraham; Tran, Thao; Xiao, Yingxian; Zarate, Carlos A.; Wainer, Irving W. Sub-anesthetic concentrations of (R,S)-ketamine metabolites inhibit acetylcholine-evoked currents in α7 nicotinic acetylcholine receptors European Journal of Pharmacology, 2013 , vol. 698, # 1-3 p. 228-234
Morris, R.G.M., 2006. Elements of a neurobiological theory of hippocampal function: the role of synaptic plasticity, synaptic tagging and schemas. Eur. J. Neurosci. 23, 2829-2846. doi:10.1111/j.1460-9568.2006.04888.x
Mullan, M., Crawford, F., Axelman, K., Houlden, H., Lilius, L., Winblad, B., Lannfelt, L., 1992. A pathogenic mutation for probable Alzheimer's disease in the APP gene at the N-terminus of beta-amyloid. Nat. Genet. 1, 345-347. doi:10.1038/ng0892-345
Nirthanan et al., Candoxin, a Novel Toxin from Bungarus candidus, Is a Reversible Antagonist of Muscle (αβγδ) but a Poorly Reversible Antagonist of Neuronal α7 nAChR Receptors JBC Vol. 277, No. 20, Issue of May 17, pp. 17811-17820, 2002
Nirthanan & Gwee, Three-Finger α-Neurotoxins and the Nicotinic Acetylcholine Receptor, Forty Years On J Pharmacol Sci 94, 1 - 17 (2004)
Nordberg, A., 2001. Nicotinic receptor abnormalities of Alzheimer's disease: therapeutic implications. Biol. Psychiatry, Nicotine Mechanisms in Alzheimer's Disease 49, 200- 210. doi:10.1016/S0006-3223(00)01125-2
Nordberg, A., Lundqvist, H., Hartvig, P., Lilja, A., Långström, B., 1995. Kinetic analysis of regional (S)(-)11C-nicotine binding in normal and Alzheimer brains--in vivo assessment using positron emission tomography. Alzheimer Dis. Assoc. Disord. 9, 21-27.
Ochoa et al. The prototoxin LYPD6B modulates heteromeric α3β4-containing nicotinic acetylcholine receptors, but not α7 homomers FASEB J. 30, 1109-1119 (2016)
S.M. O'Connor, A.A. Grishin, E. Poiraud, B. Huyard, C. Coles, Y. Kolev, S. Wagner, E. Andriambeloson The novel anxiolytic compound BNC210 is a negative allosteric modulator of the alpha 7 nicotinic acetylcholine receptor Poster Neuroscience 2014
Ohm, T.G., 2007. The dentate gyrus in Alzheimer's disease. Prog. Brain Res. 163, 723-740. doi:10.1016/S0079-6123(07)63039-8
Okada, H., Ouchi, Y., Ogawa, M., Futatsubashi, M., Saito, Y., Yoshikawa, E., Terada, T., Oboshi, Y., Tsukada, H., Ueki, T., Watanabe, M., Yamashita, T., Magata, Y., 2013. Alterations in alpha-4 beta-2 nicotinic receptors in cognitive decline in Alzheimer's aetiopathology. Brain J. Neurol. 136, 3004-3017. doi:10.1093/brain/awt195.
Orac CM, Maciagiewicz I, Bergmeier SC, McKay DB. 3D-QSAR and 3D-QSSR models of negative allosteric modulators facilitate the design of a novel selective antagonist of human α4β2 neuronal nicotinic acetylcholine receptors. Bioorg MedChem Lett. 2012 Feb 15;22(4):1797-813.
Orr-Urtreger, A., Göldner, F.M., Saeki, M., Lorenzo, I., Goldberg, L., Biasi, M.D., Dani, J.A., Patrick, J.W., Beaudet, A.L., 1997. Mice Deficient in the alpha-7 Neuronal Nicotinic Acetylcholine Receptor Lack a-Bungarotoxin Binding Sites and Hippocampal Fast Nicotinic Currents. J. Neurosci. 17, 9165-9171.
Oz M, Ravindran A, Diaz-Ruiz O, Zhang L, and Morales M (2003) The endogenous cannabinoid anandamide inhibits alpha7 nicotinic acetylcholine receptor-mediated responses in Xenopus oocytes. J Pharmacol Exp Ther 306:1003-1010
Pandya, A., Yakel, J.L., 2011. Allosteric Modulator Desformylflustrabromine Relieves the Inhibition of alpha-2 beta-2 and alpha-4 beta-2 Nicotinic Acetylcholine Receptors by beta-Amyloid1-42 Peptide. J. Mol. Neurosci. 45, 42-47. doi:10.1007/s12031-011-9509-3
Paxinos, G., Franklin, K.B.J., 2004. The Mouse Brain in Stereotaxic Coordinates. Academic Press, San Diego, CA, USA.
Paylor, R., Nguyen, M., Crawley, J.N., Patrick, J., Beaudet, A., Orr-Urtreger, A., 1998. Alpha-7 Nicotinic Receptor Subunits Are Not Necessary for Hippocampal-Dependent Learning or Sensorimotor Gating: A Behavioral Characterization of Alpha-7 -Deficient Mice. Learn. Mem. 5, 302-316. doi:10.1101/lm.5.4.302
Picciotto, M.R., Zoli, M., Léna, C., Bessis, A., Lallemand, Y., LeNovère, N., Vincent, P., Pich, E.M., Brûlet, P., Changeux, J.-P., 1995. Abnormal avoidance learning in mice lacking functional high-affinity nicotine receptor in the brain. Nature 374, 65-67. doi:10.1038/374065a0
Pouny I et al, Cytisine-like alkaloids from Ormosia hosiei Hemsl. & E.H. Wilson. Phytochemistry. 2014 Nov;107:97-101
Pudifoot et al. LY6H Regulates Trafficking of Alpha7 Nicotinic Acetylcholine Receptors and Nicotine-Induced Potentiation of Glutamatergic Signaling The Journal of Neuroscience, February 25, 2015 35(8):3420 -3430
Rabenstein et al (2006) The nicotinic antagonist mecamylamine has antidepressant-like effects in wild-type but not β2- or α7-nicotinic acetylcholine receptor subunit knockout mice. Psychopharmacol. 189 395.
Sadigh-Eteghad S, Talebi M, Mahmoudi J, Babri S, Shanehbandi D (2015). "Selective activation of α 7 nicotinic acetylcholine receptor by PHA-543613 improves Aβ 25-35-mediated cognitive deficits in mice". Neuroscience. 298: 81-93
Saviola et al. Rear-fanged snake venoms: An untapped source of novel compounds and potential drug leads Toxin Reviews, 2014, vol. 33, #4 p.185-201
Shaughnessy, L., Thomas, M.B., Wakefield, J., Chamblin, B., Nair, A., Koentgen, F., Ramabhadran, R., 2005. Lentiviral vector-based models of amyloid pathology: from cells to animals. Curr. Alzheimer Res. 2, 239-247.
Singh, Nagendra S.; Paul, Rajib K.; Ramamoorthy, Anuradha; Torjman, Marc C.; Moaddel, Ruin; Bemier, Michel; Wainer, Irving W. Nicotinic acetylcholine receptor antagonists alter the function and expression of serine racemase in PC-12 and 1321N1 cells Cellular Signalling, 2013 , vol. 25, # 12 p. 2634 - 2645
Sinkus et al, The human CHRNA7 and CHRFAM7A genes: A review of the genetics, regulation, and function. Neuropharmacology, 2015 Sep;96(Pt B):274-88
Son, J.-H., Winzer-Serhan, U.H., 2008. Expression of neuronal nicotinic acetylcholine receptor subunit mRNAs in rat hippocampal GABAergic interneurons. J. Comp. Neurol. 511, 286-299. doi:10.1002/cne.21828
Sowemimo BO, Beal JL, Doskotch RW, Svoboda GH. The isolation of stepharine and coclaurine from Sarcopetalumharveyanum. Lloydia. 1972 Mar;35(1):90-1.
Spivak CE, Lupica CR, Oz M. The endocannabinoid anandamide inhibits the function of alpha4beta2 nicotinic acetylcholine receptors. Mol Pharmacol. 2007 Oct;72(4):1024-32. Epub 2007 Jul 12
Squire, L.R., Wixted, J.T., Clark, R.E., 2007. Recognition memory and the medial temporal lobe: a new perspective. Nat. Rev. Neurosci. 8, 872-883. doi:10.1038/nrn2154
Torres et al., NMR structure of bucandin, a neurotoxin from the venom of the Malayan krait (Bungarus candidus) Biochem. J. (2001) 360, 539±548
Tsuneki H, You Y, Toyooka N, et al. (2004). "Alkaloids indolizidine 235B', quinolizidine 1-epi-2071, and the tricyclic 205B are potent and selective noncompetitive inhibitors of nicotinic acetylcholine receptors". Mol. Pharmacol. 66 (4): 1061-9.
VEYS A novel selective α7 nicotinic acetylcholine receptor allosteric modulator showing concentration dependent positive and negative allosteric modulation Program#/Poster#: 478.11/B7 Presenter at Poster: Tue, Oct. 20, 2015
Wang, H.-Y., Lee, D.H.S., D'Andrea, M.R., Peterson, P.A., Shank, R.P., Reitz, A.B., 2000a. Beta-Amyloid1-42 Binds to alpha-7 Nicotinic Acetylcholine Receptor with High Affinity Implications For Alzheimer's Disease Pathology. J. Biol. Chem. 275, 5626-5632. doi:10.1074/jbc.275.8.5626
Wang, H.-Y., Lee, D.H.S., Davis, C.B., Shank, R.P., 2000b. Amyloid Peptide Abeta1-42 Binds Selectively and with Picomolar Affinity to alpha-7 Nicotinic Acetylcholine Receptors. J. Neurochem. 75, 1155-1161. doi:10.1046/j.1471-4159.2000.0751155.x
Wang et al, The duplicated α7 subunits assemble and form functional nicotinic receptors with the full-length α7. J Biol Chem. 2014 Sep 19;289(38
Whiteaker P, Christensen S, Yoshikami D, et al. (2007). "Discovery, synthesis, and structure activity of a highly selective α7 nicotinic acetylcholine receptor antagonist". Biochemistry. 46 (22): 6628-38.
Whitehouse, P.J., Price, D.L., Struble, R.G., Clark, A.W., Coyle, J.T., Delon, M.R., 1982. Alzheimer's disease and senile dementia: loss of neurons in the basal forebrain. Science 215, 1237-1239.doi:10.1126/science.7058341
Wirths, O., Multhaup, G., Bayer, T.A., 2004. A modified beta-amyloid hypothesis: intraneuronal accumulation of the beta-amyloid peptide - the first step of a fatal cascade. J. Neurochem. 91, 513-520.doi:10.1111/j.14 71-4159.2004.02737.x
Woolf, N.J., Butcher, L.L., 2011. Cholinergic systems mediate action from movement to higher consciousness. Behav. Brain Res. 221, 488-498. doi:10.1016/j.bbr.2009.12.046
Wotring and Yoon (1995) The inhibitory effects of nicotinic antagonists on currents elicited by GABA in rat hippocampal neurons. Neurosci. 67 293.
Wu et al. Mechanisms of Inhibition and Potentiation of α4β2 Nicotinic Acetylcholine Receptors by Members of the Ly6 Protein Family The Journal Of Biological Chemistry Vol. 290, No. 40, pp.24509-24518,2015
Wu et al. SLEEPLESS is a bi-functional regulator of excitability and cholinergic synaptic transmission Curr Biol. 2014 March 17; 24(6): 621-629
Young, J.W., Crawford, N., Kelly, J.S., Kerr, L.E., Marston, H.M., Spratt, C., Finlayson, K., Sharkey, J., 2007. Impaired attention is central to the cognitive deficits observed in alpha-7 deficient mice. Eur. Neuropsychopharmacol. 17, 145-155. doi:10.1016/j.euroneuro.2006.03.008
Young, J.W., Finlayson, K., Spratt, C., Marston, H.M., Crawford, N., Kelly, J.S., Sharkey, J., 2004. Nicotine improves sustained attention in mice: evidence for involvement of the alpha-7 nicotinic acetylcholine receptor. Neuropsychopharmacol. Off. Publ. Am. Coll. Neuropsychopharmacol. 29, 891-900. doi:10.1038/sj.npp.1300393.
Young, J.W., Meves, J.M., Tarantino, I.S., Caldwell, S., Geyer, M.A., 2011. Delayed procedural learning in alpha-7-nicotinic acetylcholine receptor knockout mice. Genes Brain Behav. 10, 720-733. doi:10.1111/j.1601-183X.2011.00711.x
Yu D, Zhang L, Eisele J-L, Bertrand D, Changeux J-P, Weight F (1996) Ethanol inhibition of nicotinic acetylcholine type α.7 receptors involves the amino-terminal domain of the receptor. Mol Pharmacol 50:1010- 1016
Zanos, Panos; Moaddel, Ruin; Morris, Patrick J.; Georgiou, Polymnia; Fischell, Jonathan; Elmer, Greg I.; Alkondon, Manickavasagom; Yuan, Peixiong; Pribut, Heather J.; Singh, Nagendra S.; Dossou, Katina S. S.; Fang, Yuhong; Huang, Xi-Ping; Mayo, Cheryl L.; Wainer, Irving W.; Albuquerque, Edson X.; Thompson, Scott M.; Thomas, Craig J.; Zarate Jr, Carlos A.; Gould, Todd D. (2016). "NMDAR inhibition-independent antidepressant actions of ketamine metabolites". Nature. 533: 481-486.
Zufferey, R., Donello, J.E., Trono, D., Hope, T.J., 1999. Woodchuck Hepatitis Virus Posttranscriptional Regulatory Element Enhances Expression of Transgenes Delivered by Retroviral Vectors. J. Virol. 73, 2886-2892.

## Claims

1. A composition comprising a therapeutically effective amount of a beta-2 nicotinic acetylcholine receptor (nAChR) antagonist and/or beta-2 nAChR negative allosteric modulator (NAM), wherein said beta-2 nAChR antagonist and/or beta-2 nAChR NAM reduces acetylcholine signaling through at least one beta-2 -containing nAChR selected from the group consisting of: alpha-2 alpha-5 beta-2, alpha-2 beta-2, alpha-3 beta-2, alpha-6 beta-2, alpha-3 alpha-5 beta-2, alpha-3 alpha-6 beta-2, alpha-4 alpha-5 beta-2, alpha-4 beta-2 beta-3, and alpha-5 alpha-6 beta-2, for use to prevent or treat a subject suspected to have or suffering from Alzheimer's disease (AD).

2. A composition comprising an effective amount of a beta-2 nAChR antagonist and/or beta-2 nAChR NAM, wherein said beta-2 nAChR antagonist and/or beta-2 nAChR NAM reduces acetylcholine signaling through at least one beta-2 -containing nAChR selected from the group consisting of: alpha-2 alpha-5 beta-2, alpha-2 beta-2, alpha-3 beta-2, alpha-6 beta-2, alpha-3 alpha-5 beta-2, alpha-3 alpha-6 beta-2, alpha-4 alpha-5 beta-2, alpha-4 beta-2 beta-3, and alpha-5 alpha-6 beta-2, for use to reduce the rate of development of mild cognitive impairment and/or learning disorders and/or memory disorders in a subject suspected to have or suffering from Alzheimer's disease (AD).

3. The composition for use according to claim 1 or 2, further comprising a therapeutically effective amount of an alpha-7 nAChR antagonist and/or alpha-7 nAChR NAM

4. The composition for use according to any one of claims 1 to 3, wherein said subject is diagnosed with pre-dementia phase or dementia phase of AD

5. The composition for use according to any one of claims 1 to 4, wherein said nAChR antagonist and/or NAM is administered over at least one month, preferably over at least six months, more preferably at a regular dosing interval of from three to twenty-four hours.

6. The composition for use according to claims 1 to 5, further comprising an inhibitor of acetylcholine esterase.

7. A combination product comprising:
a) a beta-2 nAChR antagonist and/or beta-2 nAChR NAM ;
and
b) an alpha-7 nAChR antagonist and/or alpha-7 nAChR NAM,
for simultaneous, separated or staggered use for preventing Alzheimer's disease (AD) or for treating a subject suspected to have or suffering from Alzheimer's disease (AD).

8. A combination product comprising:
a) a beta-2 nAChR antagonist and/or beta-2 nAChR NAM ;
and
b) an alpha-7 nAChR antagonist and/or alpha-7 nAChR NAM,
for simultaneous, separated or staggered use for reducing the rate of development of mild cognitive impairment and/or learning disorders and/or memory disorders in a subject suspected to have or suffering from Alzheimer's disease (AD).

9. The combination product for use according to claim 7 or 8, wherein said beta-2 nAChR antagonist and/or beta-2 nAChR NAM reduces acetylcholine signaling through at least one beta-2 -containing nAChR selected from the group consisting of: alpha-2 alpha-5 beta-2, alpha-2 beta-2, alpha-3 beta-2, alpha-4 beta-2, alpha-6 beta-2, alpha-7 beta-2, alpha-3 alpha-5 beta-2, alpha-3 alpha-6 beta-2, alpha-4 alpha-5 beta-2, alpha-4 beta-2 beta-3, and alpha-5 alpha-6 beta-2.

10. The combination product for use according to any one of claims 7 to 9, wherein said beta-2 nAChR antagonist and/or beta-2 nAChR NAM reduces acetylcholine signaling through at least one beta-2 -containing nAChR selected from the group consisting of: alpha-2 alpha-5 beta-2, alpha-2 beta-2, alpha-3 beta-2, alpha-4 beta-2, alpha-6 beta-2, alpha-3 alpha-5 beta-2, alpha-3 alpha-6 beta-2, alpha-4 alpha-5 beta-2, alpha-4 beta-2 beta-3, and alpha-5 alpha-6 beta-2.

11. The combination product for use according to claim 7 or 8, wherein said beta-2 nAChR antagonist is chosen in the group consisting of: Dihydro-beta-erythroidine, Erysodine, (-)-7-methyl-2-exo-[3'-(6-[18F]fluoropyridin-2-yl)-5'-pyridinyl]-7-azabicyclo[2.2.1]heptane, 2-fluoro-3-( 4-nitro-phenyl)deschloroepibatidine, Coclaurine, Mecamylamine, and Tubocurarine; and said beta-2 nAChR NAM is chosen in the group consisting of : Amantadine, UCI-3002, COB-3, KAB-18, Lynx-1, Lynx-2, COB-1, COB-2, DDR-15, DDR-13, DDR-18, APB-8, DDR-1, DDR-2, KAB-21, DDR-19, KAB-24, KAB-22, KAB-23, KAB-19, KAB-20, KAB-30, IB-2, PPB-12, PPB-13, IB-4, COB-4, LYPD6, LYPD6B, PSCA, PATE-M, SLURP1, SLURP2, ly6A/E, ly6B-G, Ly6G6E, ly6H, SLEEPLESS, Odr-2, Boudin, Nl-lynx1, Nl-lynx2, Pr-lynx1, Erabutoxin b, Bucandin, Candoxin, Fasciculins, and alpha-colubritoxin.

12. The combination product for use according to any one of claim 7 to 11, wherein said alpha-7 nAChR antagonist is chosen in the group consisting of ethanol, hydroxynorketamine, ketamine, kynurenic acid, lobeline, methyllycaconitine (MLA), Norketamine, Quinolizidine (-)-1-epi-207I, S76892, dizocilpine (+)-MK-801, and/or alpha-7 nAChR NAM is chosen in the group consisting of: 1,2,3,3a,4,8b-hexahydro-2-benzyl-6-N,N-dimethylamino-1-methylindeno[1,2,-b]pyrrole (HDMP), 2,3,6 MP-TQS, cistrans-4-(2,3,6-trimethylphenyl)-3a,4,5,9b-tetrahydro-3H-cyclopenta[c]quinoline-8-sulfonamide, 2,6MP-TQS, cis-trans-4-(2,6-dimethylphenyl)-3a,4,5,9b-tetrahydro-3H cyclopenta[c]quinoline-8-sulfonamide, BNC210, EVP-6124, Methanandamide.

13. The combination product for use according to any one of claims 7 to 12, further comprising a therapeutically effective amount of an inhibitor of acetylcholine esterase.

14. The combination product for use according to any one of claims 7 to 13, wherein said subject is diagnosed with pre-dementia phase or dementia phase of AD.

15. The combination product for use according to any one of claims 7 to 14, wherein said nAChR antagonist and/or NAM is administered over at least one month, preferably over at least six months, more preferably at a regular dosing interval of from three to twenty-four hours.

## Patentansprüche

1. Zusammensetzung, die eine therapeutisch wirksame Menge eines Antagonisten eines beta-2 nikotinischen Acetylcholinrezeptors (nAChR) und/oder eines negativen allosterischen Modulators (NAM) eines beta-2 nAChR umfasst, wobei der beta-2 nAChR-Antagonist und/oder beta-2 nAChR NAM Acetylcholin-Signalisierung durch mindestens einen beta-2-haltigen nAChR vermindert, der aus der Gruppe ausgewählt ist, die besteht aus: alpha-2 alpha-5 beta-2, alpha-2 beta-2, alpha-3 beta-2, alpha-6 beta-2, alpha-3 alpha-5 beta-2, alpha-3 alpha-6 beta-2, alpha-4 alpha-5 beta-2, alpha-4 beta-2 beta-3 und alpha-5 alpha-6 beta-2, zur Verwendung bei der Prävention oder Behandlung einer Person, bei der vermutet wird, dass sie die Alzheimer-Krankheit (AD) hat oder daran leidet.

2. Zusammensetzung, die eine wirksame Menge eines beta-2 nAChR-Antagonisten und/oder beta-2 nAChR NAM umfasst, wobei der beta-2 nAChR-Antagonist und/oder beta-2 nAChR NAM Acetylcholin-Signalisierung durch mindestens einen beta-2-haltigen nAChR vermindert, der ausgewählt ist aus der Gruppe, die besteht aus: alpha-2 alpha-5 beta-2, alpha-2 beta-2, alpha-3 beta-2, alpha-6 beta-2, alpha-3 alpha-5 beta-2, alpha-3 alpha-6 beta-2, alpha-4 alpha-5 beta-2, alpha-4 beta-2 beta-3 und alpha-5 alpha-6 beta-2, zur Verwendung bei der Verlangsamung der Geschwindigkeit der Entwicklung leichter kognitiver Beeinträchtigung und/oder Lernstörungen und/oder Gedächtnisstörungen bei einer Person, bei der vermutet wird, dass sie die Alzheimer-Krankheit (AD) hat oder daran leidet.

3. Zusammensetzung zur Verwendung nach Anspruch 1 oder 2, die ferner eine therapeutisch wirksame Menge eines alpha-7 nAChR-Antagonisten und/oder alpha-7 nAChR NAM umfasst.

4. Zusammensetzung zur Verwendung nach einem der Ansprüche 1 bis 3, wobei bei der Person die Prädemenzphase oder Demenzphase der AD diagnostiziert wurde.

5. Zusammensetzung zur Verwendung nach einem der Ansprüche 1 bis 4, wobei der nAChR-Antagonist und/oder NAM über mindestens einen Monat, vorzugsweise über mindestens sechs Monate, mehr zu bevorzugen in einem regelmäßigen Dosierungsintervall von drei bis vierundzwanzig Stunden, verabreicht wird.

6. Zusammensetzung zur Verwendung nach Anspruch 1 bis 5, die ferner einen Acetylcholinesterase-Hemmer umfasst.

7. Kombinationsprodukt, das Folgendes umfasst:
a) einen beta-2 nAChR-Antagonisten und/oder beta-2 nAChR NAM;
und
b) einen alpha-7 nAChR-Antagonisten und/oder alpha-7 nAChR NAM,
zur gleichzeitigen, getrennten oder versetzten Verwendung bei der Prävention von Alzheimer-Krankheit (AD) oder zur Behandlung einer Person, bei der vermutet wird, dass sie an Alzheimer-Krankheit (AD) leidet.

8. Kombinationsprodukt, das Folgendes umfasst:
a) einen beta-2 nAChR-Antagonisten und/oder beta-2 nAChR NAM;
und
b) einen alpha-7 nAChR-Antagonisten und/oder alpha-7 nAChR NAM,
zur gleichzeitigen, getrennten oder versetzten Verwendung bei der Verlangsamung der Geschwindigkeit der Entwicklung von leichter kognitiver Beeinträchtigung und/oder Lernstörungen und/oder Gedächtnisstörungen bei einer Person, bei der vermutet wird, dass sie die Alzheimer-Krankheit (AD) hat oder daran leidet.

9. Kombinationsprodukt zur Verwendung nach Anspruch 7 oder 8, wobei der beta-2 nAChR-Antagonist und/oder beta-2 nAChR NAM Acetylcholin-Signalisierung durch mindestens einen beta-2-haltigen nAChR vermindert, der ausgewählt ist aus der Gruppe, die besteht aus: alpha-2 alpha-5 beta-2, alpha-2 beta-2, alpha-3 beta-2, alpha-4 beta-2, alpha-6 beta-2, alpha-7 beta-2, alpha-3 alpha-5 beta-2, alpha-3 alpha-6 beta-2, alpha-4 alpha-5 beta-2, alpha-4 beta-2 beta-3 und alpha-5 alpha-6 beta-2.

10. Kombinationsprodukt zur Verwendung nach einem der Ansprüche 7 bis 9, wobei der beta-2 nAChR-Antagonist und/oder beta-2 nAChR NAM Acetylcholin-Signalisierung durch mindestens einen beta-2-haltigen nAChR vermindert, der ausgewählt ist aus der Gruppe, die besteht aus: alpha-2 alpha-5 beta-2, alpha-2 beta-2, alpha-3 beta-2, alpha-4 beta-2, alpha-6 beta-2, alpha-3 alpha-5 beta-2, alpha-3 alpha-6 beta-2, alpha-4 alpha-5 beta-2, alpha-4 beta-2 beta-3 und alpha-5 alpha-6 beta-2.

11. Kombinationsprodukt zur Verwendung nach Anspruch 7 oder 8, wobei der beta-2 nAChR-Antagonist ausgewählt ist aus der Gruppe die besteht aus: Dihydro-beta-Erythroidin, Erysodin, (-)-7-Methyl-2-exo-[3'-(6-[18F]fluoropyridin-2-yl)-5'-pyridinyl]-7-azabicyclo[2.2.1]heptan, 2-Fluor-3-(4-nitrophenyl)deschloroepibatidin, Coclaurin, Mecamylamin und Tubocurarin; und der beta-2 nAChR NAM ausgewählt ist aus der Gruppe, die besteht aus: Amantadin, UCI-3002, COB-3, KAB-18, Lynx-1, Lynx-2, COB-1, COB-2, DDR-15, DDR-13, DDR-18, APB-8, DDR-1, DDR-2, KAB-21, DDR-19, KAB-24, KAB-22, KAB-23, KAB-19, KAB-20, KAB-30, IB-2, PPB-12, PPB-13, IB-4, COB-4, LYPD6, LYPD6B, PSCA, PATE-M, SLURP1, SLURP2, ly6A/E, ly6B-G, Ly6G6E, ly6H, SLEEPLESS, Odr-2, Boudin, Nl-lynx1, Nl-lynx2, Pr-lynx1, Erabutoxin b, Bucandin, Candoxin, Fasciculins und alpha-Colubritoxin.

12. Kombinationsprodukt zur Verwendung nach einem der Ansprüche 7 bis 11, wobei der alpha-7 nAChR-Antagonist ausgewählt ist aus der Gruppe, die besteht aus: Ethanol, Hydroxynorketamin, Ketamin, Kynurensäure, Lobelin, Methyllycaconitin (MLA), Norketamin, Quinolizidin(-)-1-epi-207I, S76892, Dizocilpin (+)-MK-801 und/oder der alpha-7 nAChR NAM ausgewählt ist aus der Gruppe, die besteht aus: 1,2,3,3a,4,8b-Hexahydro-2-benzyl-6-N,N-dimethylamino-1-methylinden[1,2,-b]pyrrol (HDMP), 2,3,6 MP-TQS, Cistrans-4-(2,3,6-trimethylphenyl)-3a,4,5,9b-tetrahydro-3H-cyclopenta[c]quinolin-8-sulfonamid, 2,6MP-TQS, cis-trans-4-(2,6-Dimethylphenyl)-3a,4,5,9b-tetrahydro-3H cyclopenta[c]quinolin-8-sulfonamid, BNC210, EVP-6124, Methanandamid.

13. Kombinationsprodukt zur Verwendung nach einem der Ansprüche 7 bis 12, das ferner eine therapeutisch wirksame Menge eines Acetylcholinesterase-Hemers umfasst.

14. Kombinationsprodukt zur Verwendung nach einem der Ansprüche 7 bis 13, wobei bei der Person die Prädemenzphase oder Demenzphase der AD diagnostiziert wurde.

15. Kombinationsprodukt zur Verwendung nach einem der Ansprüche 7 bis 14, wobei der nAChR-Antagonist und/oder NAM über mindestens einen Monat, vorzugsweise über mindestens sechs Monate, mehr zu bevorzugen in einem regelmäßigen Dosierungsintervall von drei bis vierundzwanzig Stunden, verabreicht wird.

## Revendications

1. Composition comprenant une quantité efficace du point de vue thérapeutique d'un antagoniste de récepteur d'acétylcholine nicotinique (nAChR) bêta-2 et/ou d'un modulateur allostérique négatif (NAM) de bêta-2 nAChR , dans laquelle ledit antagoniste de bêta-2 nAChR et/ou NAM de bêta-2 nAChR réduit la signalisation de l'acétylcholine par l'intermédiaire d'au moins un nAChR contenant bêta-2 choisi dans le groupe constitué par : alpha-2 alpha-5 bêta-2, alpha-2 bêta-2, alpha-3 bêta-2, alpha-6 bêta-2, alpha-3 alpha-5 bêta-2, alpha-3 alpha-6 bêta-2, alpha-4 alpha-5 bêta-2, alpha-4 bêta-2 bêta-3, et alpha-5 alpha-6 bêta-2, pour une utilisation afin de prévenir ou traiter un sujet suspecté d'avoir ou souffrant de la maladie d'Alzheimer (MA).

2. Composition comprenant une quantité efficace d'un antagoniste de bêta-2 nAChR et/ou d'un NAM de bêta-2 nAChR, dans laquelle ledit antagoniste de bêta-2 nAChR et/ou NAM de bêta-2 nAChR réduit la signalisation de l'acétylcholine par l'intermédiaire d'au moins un nAChR contenant bêta-2 choisi dans le groupe constitué par : alpha-2 alpha-5 bêta-2, alpha-2 bêta-2, alpha-3 bêta-2, alpha-6 bêta-2, alpha-3 alpha-5 bêta-2, alpha-3 alpha-6 bêta-2, alpha-4 alpha-5 bêta-2, alpha-4 bêta-2 bêta-3, et alpha-5 alpha-6 bêta-2, pour une utilisation afin de réduire la vitesse de développement d'un trouble cognitif léger et/ou de troubles de l'apprentissage et/ou de troubles de la mémoire chez un sujet suspecté d'avoir ou souffrant de la maladie d'Alzheimer (MA).

3. Composition pour une utilisation selon la revendication 1 ou 2, comprenant en outre une quantité efficace du point de vue thérapeutique d'un antagoniste de alpha-7 nAChR et/ou d'un NAM de alpha-7 nAChR.

4. Composition pour une utilisation selon l'une quelconque des revendications 1 à 3, dans laquelle ledit sujet est diagnostiqué avec une phase de pré-démence ou une phase de démence de la MA.

5. Composition pour une utilisation selon l'une quelconque des revendications 1 à 4, dans laquelle ledit antagoniste et/ou NAM de nAChR est administré sur au moins un mois, de préférence sur au moins six mois, mieux encore à intervalles d'administration réguliers de trois à vingt-quatre heures.

6. Composition pour une utilisation selon l'une quelconque des revendications 1 à 5, comprenant en outre un inhibiteur d'acétylcholine estérase.

7. Produit de combinaison comprenant :
a) un antagoniste de bêta-2 nAChR et/ou un NAM de bêta-2 nAChR;
et
b) un antagoniste de alpha-7 nAChR et/ou un NAM de alpha-7 nAChR,
pour une utilisation simultanée, séparée ou échelonnée afin de prévenir la maladie d'Alzheimer (MA) ou afin de traiter un sujet suspecté d'avoir ou souffrant de la maladie d'Alzheimer (MA).

8. Produit de combinaison comprenant :
a) un antagoniste de bêta-2 nAChR et/ou un NAM de bêta-2 nAChR;
et
b) un antagoniste de alpha-7 nAChR et/ou un NAM de alpha-7 nAChR,
pour une utilisation simultanée, séparée ou échelonnée afin de réduire la vitesse de développement d'un trouble cognitif léger et/ou de troubles de l'apprentissage et/ou de troubles de la mémoire chez un sujet suspecté d'avoir ou souffrant de la maladie d'Alzheimer (MA).

9. Produit de combinaison pour une utilisation selon la revendication 7 ou 8, dans lequel ledit antagoniste de bêta-2 nAChR et/ou NAM de bêta-2 nAChR réduit la signalisation de l'acétylcholine par l'intermédiaire d'au moins un nAChR contenant bêta-2 choisi dans le groupe constitué par : alpha-2 alpha-5 bêta-2, alpha-2 bêta-2, alpha-3 bêta-2, alpha-4 bêta-2, alpha-6 bêta-2, alpha-7 bêta-2, alpha-3 alpha-5 bêta-2, alpha-3 alpha-6 bêta-2, alpha-4 alpha-5 bêta-2, alpha-4 bêta-2 bêta-3, et alpha-5 alpha-6 bêta-2.

10. Produit de combinaison pour une utilisation selon l'une quelconque des revendications 7 à 9, dans lequel ledit antagoniste de bêta-2 nAChR et/ou NAM de bêta-2 nAChR réduit la signalisation de l'acétylcholine par l'intermédiaire d'au moins un nAChR contenant bêta-2 choisi dans le groupe constitué par : alpha-2 alpha-5 bêta-2, alpha-2 bêta-2, alpha-3 bêta-2, alpha-4 bêta-2, alpha-6 bêta-2, alpha-3 alpha-5 bêta-2, alpha-3 alpha-6 bêta-2, alpha-4 alpha-5 bêta-2, alpha-4 bêta-2 bêta-3, et alpha-5 alpha-6 bêta-2.

11. Produit de combinaison pour une utilisation selon la revendication 7 ou 8, dans lequel ledit antagoniste de bêta-2 nAChR est choisi dans le groupe constitué par : la dihydro-bêta-érythroïdine, l'érysodine, le (-)-7-méthyl-2-exo-[3'-(6-[18F]fluoropyridin-2-yl)-5'-pyridinyl]-7-azabicyclo[2.2.1]heptane, la 2-fluoro-3-(4-nitrophényl)deschloroépibatidine, la coclaurine, la mécamylamine, et la tubocurarine ; et ledit NAM de bêta-2 nAChR est choisi dans le groupe constitué par : l'amantadine, UCI-3002, COB-3, KAB-18, Lynx-1, Lynx-2, COB-1, COB-2, DDR-15, DDR-13, DDR-18, APB-8, DDR-1, DDR-2, KAB-21, DDR-19, KAB-24, KAB-22, KAB-23, KAB-19, KAB-20, KAB-30, IB-2, PPB-12, PPB-13, IB-4, COB-4, LYPD6, LYPD6B, PSCA, PATE-M, SLURP1, SLURP2, ly6A/E, ly6B-G, Ly6G6E, ly6H, SLEEPLESS, Odr-2, Boudin, Nl-lynx1, Nl-lynx2, Pr-lynx1, l'érabutoxine b, la bucandine, la candoxine, les fasciculines, et l'alpha-colubritoxine.

12. Produit de combinaison pour une utilisation selon l'une quelconque des revendications 7 à 11, dans lequel ledit antagoniste de alpha-7 nAChR est choisi dans le groupe constitué par : l'éthanol, l'hydroxynorkétamine, la kétamine, l'acide kynurénique, la lobéline, la méthyl-lycaconitine (MLA), la norkétamine, la quinolizidine (-)-1-épi-207I, S76892, la dizocilpine (+)-MK-801, et/ou le NAM de alpha-7 nAChR est choisi dans le groupe constitué par : le 1,2,3,3a,4,8b-hexahydro-2-benzyl-6-N,N-diméthylamino-1-méthylindéno[1,2,-b]pyrrole (HDMP), 2,3,6 MP-TQS, le cistrans-4-(2,3,6-triméthylphényl)-3a,4,5,9b-tétrahydro-3H-cyclopenta[c]quinoline-8-sulfonamide, 2,6MP-TQS, le cis-trans-4-(2,6-diméthylphényl)-3a,4,5,9b-tétrahydro-3H-cyclopenta[c]quinoline-8-sulfonamide, BNC210, EVP-6124, le méthanandamide.

13. Produit de combinaison pour une utilisation selon l'une quelconque des revendications 7 à 12, comprenant en outre une quantité efficace du point de vue thérapeutique d'un inhibiteur d'acétylcholine estérase.

14. Produit de combinaison pour une utilisation selon l'une quelconque des revendications 7 à 13, dans lequel ledit sujet est diagnostiqué avec une phase de pré-démence ou une phase de démence de la MA.

15. Produit de combinaison pour une utilisation selon l'une quelconque des revendications 7 à 14, dans lequel ledit antagoniste et/ou NAM de nAChR est administré sur au moins un mois, de préférence sur au moins six mois, mieux encore à intervalles d'administration réguliers de trois à vingt-quatre heures.
